# EUROPEAN PATENT APPLICATION

(11) **EP 4 817 571 A2**
(43) Date of publication of application: **07.10.2026**
(21) Application number: 25211945.8
(22) Date of filing: 31.07.2019
(51) Int. Cl.: A61F 7/10

(54) **METHODS, DEVICES, AND SYSTEMS FOR IMPROVING SKIN CHARACTERISTICS**

(30) Priority: 31.07.2018 US 201862712562 P
(62) Document number(s) of the earlier application(s) in accordance with Art. 76 EPC: 19765574.9 / 3 829 496
(71) Applicant: Zeltiq Aesthetics, Inc., Pleasanton, CA 94588 (US)
(72) Inventor: JIMENEZ LOZANO, Joel N., Pleasanton, California 94588 (US); ZENG, Like, Pleasanton, California 94588 (US); FRANGINEAS, George Jr., Fremont, California 94539 (US); PHAM, Linda, Kensington, California 94707 (US)
(74) Representative: Hoffmann Eitle

(57) **Abstract**

Provided herein are systems, compositions, and methods for improving one or more skin characteristics in a subject. These systems, compositions, and methods are configured to cool the subject's skin at a target site to a degree that alters adipocyte signaling but does not produce significant destruction of subcutaneous lipid-rich cells. In some embodiments, alteration of adipocyte signaling produces an improvement in one or more skin characteristics.

## Description

### CROSS-REFERENCE TO RELATED APPLICATIONS

The present international application claims the benefit of and priority to U.S. Provisional Patent Application No. 62/712,562, filed July 31, 2018, which is incorporated herein by reference in its entirety.

### BACKGROUND

Skin is made up of a surface epidermis layer and a thicker dermal layer immediately below the epidermis. A hypodermis area, also known as a subcutaneous layer, lies immediately below the dermis. This subcutaneous fat layer stores fat and serves to anchor the dermis to underlying muscles and bones.

Cryolipolysis is a non-invasive method for destroying lipid-rich cells (e.g., adipocytes) in subcutaneous fat by cooling target tissue in a controlled manner to reduce a volume of the fat and result in a slimmer aesthetically pleasing appearance. Cold temperatures are applied to the epidermis to cool the subcutaneous layer to a target temperature for a period of time sufficient to damage lipid-rich cells (e.g., adipocytes). These cells are then degraded and the lipids are removed over time by the body.

Cryolipolytic fat removal requires the temperature of the subcutaneous fat layer to be lowered to a sufficiently low temperature for a sufficiently long period of time to damage significant numbers of fat cells. A variety of specific protocols have been developed for achieving this. Generally, lipid-rich target tissue (e.g., subcutaneous fat) is lowered from a temperature of about 10°C to about -25°C for an interval of about 10 seconds to 30 minutes (see, e.g., U.S. Patent No. 7,367,341). In certain protocols, multiple cooling cycles are utilized over the course of a single treatment session, with cooling cycles separated by non-cooling cycles. Treatment sessions may be repeated several times over the course of days, weeks, or months.

Cold treatment can affect and damage fat cells and non-fat cells under certain conditions. Therefore, one factor limiting the application of cryolipolysis is the potential for damage to the surrounding epidermis due to overexposure to cold temperatures. For this reason, fat removal protocols generally seek to limit exposure time and/or keep temperatures above certain thresholds to prevent or minimize damage to non-fat cells.

### SUMMARY

Provided herein in certain embodiments are methods of improving one or more skin characteristics in a subject comprising cooling the subject's skin at a target site to a degree that alters adipocyte signaling but does not produce significant destruction of subcutaneous lipid-rich cells, wherein said alteration of adipocyte signaling produces an improvement in one or more skin characteristics. In certain embodiments, less than 10% of the subcutaneous lipid-rich cells are destroyed. In certain embodiments, less than 1%, 2%, 3%, 4%, 5%, or 7% of the subcutaneous lipid-rich cells are destroyed. In some embodiments, said cooling does not produce any adverse skin effects. In certain embodiments, said adverse effects are selected from the group consisting of hyper-pigmentation, hypo-pigmentation, unwanted blistering, unwanted scarring, permanent undesirable alterations, and disfiguring scars. In certain embodiments, said alteration in adipocyte signaling results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3. In some embodiments, said increase in expression occurs in the dermal layer, the subcutaneous layer, or both. In certain embodiments, said alteration in adipocyte signaling results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases. In some embodiments, increase in one or more extracellular matrix components occurs in the epidermal layer, dermal layer, the subcutaneous layer, or combinations thereof. In certain embodiments, said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.

Provided herein in certain embodiments are methods of improving one or more skin characteristics in a subject comprising cooling the subject's skin at a target site to a degree that alters adipocyte signaling but does not produce significant destruction of subcutaneous lipid-rich cells, wherein said alteration of adipocyte signaling produces an improvement in one or more skin characteristics. In certain embodiments, said cooling is performed by applying a treatment unit proximal to the target site. In certain embodiments, the temperature of said treatment unit is about -18°C to about 0°C. In some embodiments, said cooling lowers the temperature of the epidermis at the target site to about -15°C to about 5°C. In certain embodiments, said cooling is discontinued after the temperature of the epidermis at the target site has been at a temperature of about -15°C to about 5°C for about 10 minutes to about 25 minutes. In certain embodiments, said cooling does not lower the temperature of the subcutaneous fat layer 7 mm below the target site below about 3°C. In some embodiments, said cooling lowers the temperature of the subcutaneous fat layer 7 mm below the target site to about 3°C to about 30°C. In certain embodiments, said cooling is discontinued after the temperature of the subcutaneous fat layer 7 mm below the target site has been at a temperature of about 3°C to about 30°C for about 10 minutes to about 25 minutes. In some embodiments, said cooling is discontinued before the temperature of the subcutaneous fat layer 7 mm below the target site falls below 3°C. In certain embodiments, said cooling is repeated two or more times separated by re-warming periods during a single treatment session.

Provided herein in certain embodiments are methods of improving one or more skin characteristics in a subject comprising applying a cooling element proximal to a target site on the subject's skin for a period of time sufficient to cool the epidermis at the target site to about -15°C to about 5°C, wherein said cooling results in an alteration of one or more adipocyte signaling events; and removing the cooling element before the temperature of the subcutaneous fat layer about 7 mm below the target site decreases below a temperature of +3C. In certain embodiments, the temperature of the subcutaneous fat layer about 7 mm below the target site is decreased to about 3°C to about 15°C during application of the cooling element. In certain embodiments, less than 10% of the subcutaneous lipid-rich cells in the entire subcutaneous fat layer are destroyed. In some embodiments, less than either 1%, 2%, 3%, 4%, 5%, or 7% of the subcutaneous lipid-rich cells are destroyed.

Provided herein in certain embodiments are methods of improving one or more skin characteristics in a subject comprising applying a cooling element proximal to a target site on the subject's skin for a period of time sufficient to cool the epidermis at the target site to about -15°C to about 5°C, wherein said cooling results in an alteration of one or more adipocyte signaling events; and removing the cooling element before the temperature of the entire subcutaneous fat layer beneath the target site is decreased to a level that produces significant destruction of subcutaneous lipid-rich cells therein. In certain embodiments, the temperature of the subcutaneous fat layer about 7 mm below the target site is decreased to about 3°C to about 15°C during application of the cooling element. In certain embodiments, less than 10% of the subcutaneous lipid-rich cells in the entire subcutaneous fat layer are destroyed. In some embodiments, less than either 1%, 2%, 3%, 4%, 5%, or 7% of the subcutaneous lipid-rich cells are destroyed.

Provided herein in certain embodiments are methods of increasing new collagen formation in a subject comprising cooling the subject's skin at a target site to a degree that alters adipocyte signaling but does not produce significant destruction of subcutaneous lipid-rich cells, wherein said alteration of adipocyte signaling increases new collagen formation. Also provided herein in certain embodiments are methods of increasing new collagen formation in a subject comprising applying a cooling element proximal to a target site on the subject's skin for a period of time sufficient to cool the epidermis at the target site to about -15°C to about 5°C, wherein said cooling results in an alteration of one or more adipocyte signaling events and an increase in new collagen formation; and removing the cooling element before the temperature of the subcutaneous fat layer about 7 mm below the target site decreases below a temperature of +3C. Further provided herein in certain embodiments are methods of increasing new collagen formation in a subject comprising applying a cooling element proximal to a target site on the subject's skin for a period of time sufficient to cool the epidermis at the target site to about -15°C to about 5°C, wherein said cooling results in alteration of adipocyte signaling and an increase in new collagen formation; and removing the cooling element before the temperature of the entire subcutaneous fat layer beneath the target site is decreased to a level that produces significant destruction of subcutaneous lipid-rich cells therein. In certain embodiments, collagen formation is increased in the dermal fat layer. In certain embodiments, collagen formation is increased in the basal epidermal junction (e.g., attaches the basal lamina to the dermis), dermis, and fibrous septae.

Provided herein in certain embodiments are methods of decreasing skin laxity in a subject comprising cooling the subject's skin at a target site to a degree that alters adipocyte signaling but does not produce significant destruction of subcutaneous lipid-rich cells, wherein said alteration of adipocyte signaling decreases skin laxity. Also provided herein in certain embodiments are methods of decreasing skin laxity in a subject comprising applying a cooling element proximal to a target site on the subject's skin for a period of time sufficient to cool the epidermis at the target site to about -15°C to about 5°C, wherein said cooling results in an alteration of one or more adipocyte signaling events and a decrease in skin laxity; and removing the cooling element before the temperature of the subcutaneous fat layer about 7 mm below the target site decreases below a temperature of +3C. Further provided herein in certain embodiments are methods of decreasing skin laxity in a subject comprising applying a cooling element proximal to a target site on the subject's skin for a period of time sufficient to cool the epidermis at the target site to about -15°C to about 5°C, wherein said cooling results in an alteration of one or more adipocyte signaling events and a decrease in skin laxity; and removing the cooling element before the temperature of the entire subcutaneous fat layer beneath the target site is decreased to a level that produces significant destruction of subcutaneous lipid-rich cells therein.

Provided herein in certain embodiments are methods of increasing skin thickness comprising cooling the subject's skin at a target site to a degree that alters adipocyte signaling but does not produce significant destruction of subcutaneous lipid-rich cells, wherein said alteration of adipocyte signaling produces an increase in skin thickness. Also provided herein in certain embodiments are methods of increasing skin thickness in a subject comprising applying a cooling element proximal to a target site on the subject's skin for a period of time sufficient to cool the epidermis at the target site to about -15°C to about 5°C, wherein said cooling results in an alteration of one or more adipocyte signaling events and an increase in skin thickness; and removing the cooling element before the temperature of the subcutaneous fat layer about 7 mm below the target site decreases below a temperature of +3C. Further provided herein in certain embodiments are methods of increasing skin thickness in a subject comprising applying a cooling element proximal to a target site on the subject's skin for a period of time sufficient to cool the epidermis at the target site to about -15°C to about 5°C, wherein said cooling results in an alteration of one or more adipocyte signaling events and an increase in skin thickness; and removing the cooling element before the temperature of the entire subcutaneous fat layer beneath the target site is decreased to a level that produces significant destruction of subcutaneous lipid-rich cells therein.

Provided herein in certain embodiments are systems for use in the methods disclosed herein. Also provided herein in some embodiments are systems for improving one or more skin characteristics in a subject, comprising a treatment unit; and an applicator having a cooling unit in communication with the treatment unit, wherein the applicator is configured to cool the subject's skin at a target site to a degree that alters adipocyte signaling but does not produce significant destruction of subcutaneous lipid-rich cells. In some embodiments, a temperature of said treatment unit is about -18°C to about 0°C. In certain embodiments, when said applicator cools the subject's skin, said cooling lowers the temperature of an epidermis at the target site to about -15°C to about 5°C. In certain embodiments, when said applicator cools the subject's skin, said cooling is discontinued after the temperature of the epidermis at the target site has been at a temperature of about -15°C to about 5°C for about 10 minutes to about 25 minutes. In some embodiments, when said applicator cools the subject's skin, said cooling does not lower the temperature of the subcutaneous fat layer 7 mm below the target site below about 3°C. In certain embodiments, when said applicator cools the subject's skin, said cooling lowers the temperature of the subcutaneous fat layer 7 mm below the target site to about 3°C to about 30°C. In certain embodiments, when said applicator cools the subject's skin, said cooling is discontinued after the temperature of the subcutaneous fat layer 7 mm below the target site has been at a temperature of about 3°C to about 30°C for about 10 minutes to about 25 minutes. In some embodiments, when said applicator cools the subject's skin, said cooling is discontinued before the temperature of the subcutaneous fat layer 7 mm below the target site falls below 3°C. In certain embodiments, when said applicator cools the subject's skin, said cooling is repeated two or more times separated by re-warming periods during a single treatment session. In some embodiments, when the applicator alters adipocyte signaling, an improvement in one or more skin characteristics is produced.

Provided herein in some embodiments are systems for improving one or more skin characteristics in a subject, comprising a treatment unit; and an applicator having a cooling unit in communication with the treatment unit, wherein the applicator is configured to cool the subject's skin at a target site to a degree that alters adipocyte signaling but does not produce significant destruction of subcutaneous lipid-rich cells. In some embodiments, less than 10% of the subcutaneous lipid-rich cells are destroyed. In certain embodiments, less than 1%, 2%, 3%, 4%, 5%, or 7% of the subcutaneous lipid-rich cells are destroyed. In certain embodiments, when the applicator cools the subject's skin at the target site, the cooling does not produce any adverse skin effects. In some embodiments, said adverse effects are selected from the group consisting of hyper-pigmentation, hypo-pigmentation, unwanted blistering, unwanted scarring, permanent undesirable alterations, and disfiguring scars. In certain embodiments, when the applicator alters adipocyte signaling, said alteration results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3. In some embodiments, when the applicator alters adipocyte signaling, said increase in expression occurs in the dermal layer, the subcutaneous layer, or both. In certain embodiments, when the applicator alters adipocyte signaling, said alteration results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases. In certain embodiments, when the applicator alters adipocyte signaling, said increase in one or more extracellular matrix components occurs in the epidermal layer, dermal layer, the subcutaneous layer, or combinations thereof. In certain embodiments, when the applicator alters adipocyte signaling, said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.

### BRIEF DESCRIPTION OF DRAWINGS

The patent or application file contains at least one drawing executed in color. Copies of this patent or patent application publication with color drawing(s) will be provided by the Office upon request and payment of the necessary fee.

In the drawings, identical reference numbers identify similar elements or acts. The sizes and relative positions of elements in the drawings are not necessarily drawn to scale. For example, the shapes of various elements and angles may not be drawn to scale, and some of these elements are arbitrarily enlarged and positioned to improve drawing legibility. Further, the particular shapes of the elements as drawn are not intended to convey any information regarding the actual shape of the particular elements, and have been solely selected for ease of recognition in the drawings.

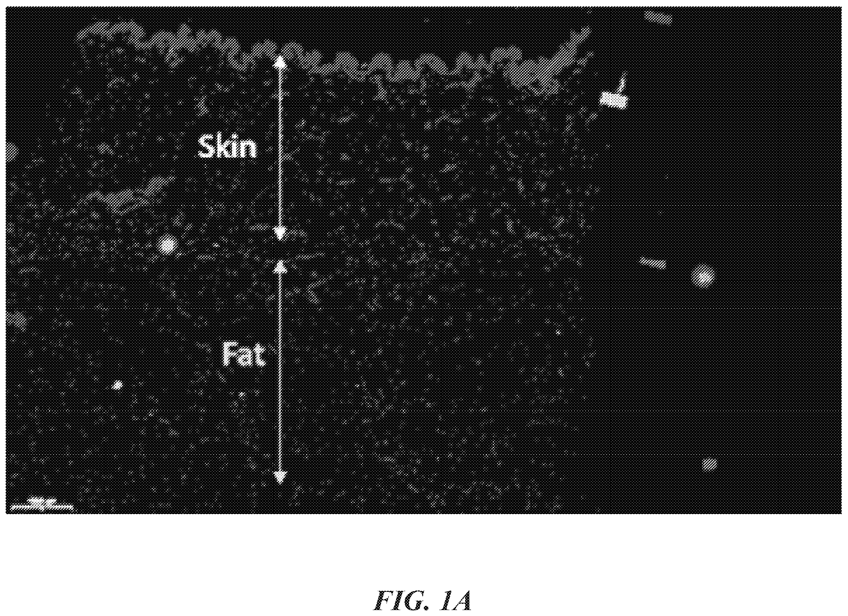

FIGS. 1A-1D: Representative tissue sections showing the effect of controlled epidermal cooling on TGF-β mRNA expression in skin and adipose tissue visualized by in situ hybridization. Fluorescence pseudocolor: TGF-β mRNA = Cy5, yellow. Nucleus = TRITC, blue. 1A: Control (untreated tissue) with no change in TGF-β mRNA expression in skin and fat tissue. 1B-1D: 3 weeks post-treatment. 1B: Slide showing a strong signal of Cy5 representing elevated expression of TGF-β mRNA in dermal and subcutaneous adipose tissue post-treatment. 1C and 1D: Magnifications of dermal and subcutaneous fat, respectively, showing elevated expression of TGF-β mRNA around adipocytes (arrows).

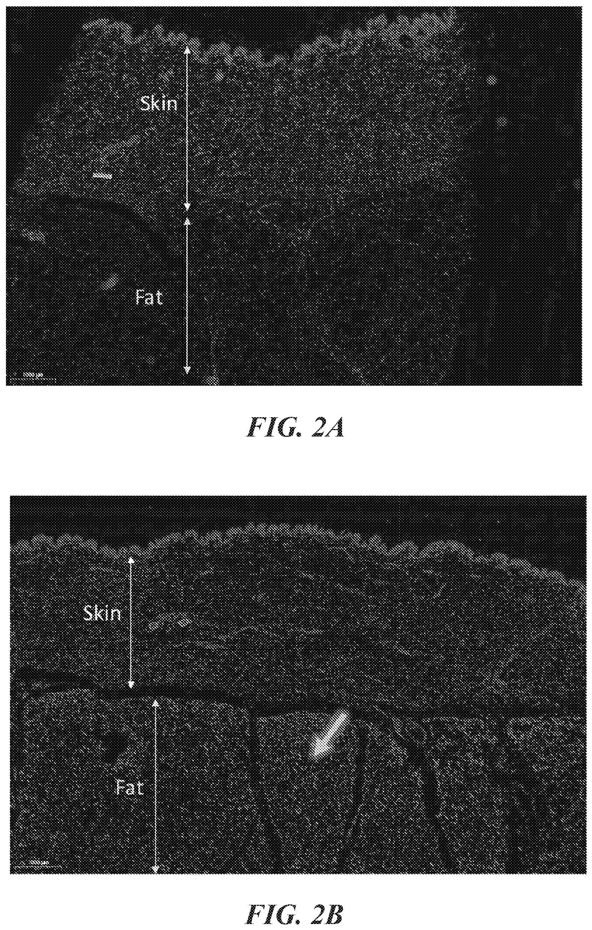

FIGS. 2A-2B: Representative tissue sections showing the effect of controlled epidermal cooling on collagen COL1A1 mRNA expression in skin and adipose tissue visualized by in situ hybridization. Fluorescence pseudocolor: COL1A1 mRNA = Cy5, red. Nucleus = TRITC, blue. 2A: Control (untreated tissue), showing positive signal for Cy5 only in skin and collagenous structures representing expression of COL1A1 mRNA. 2B: 3 weeks post-treatment sample showing an elevated signal of COL1A1 mRNA expression in subcutaneous adipose tissue.

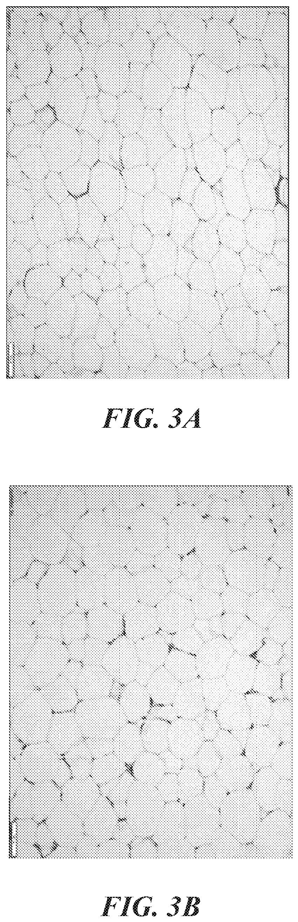

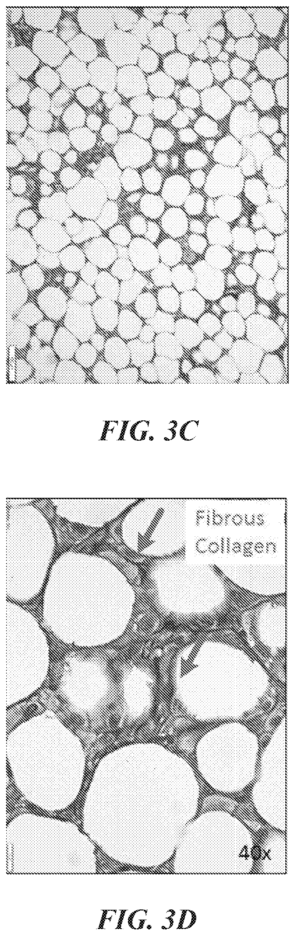

FIGS. 3A-3D: Representative adipose tissue sections showing collagen synthesis near adipocytes following controlled epidermal cooling. Collagen = Masson's Trichrome, blue. 3A: Control (untreated), showing no collagen staining around adipocytes. 3B: 1-week post-treatment, no collagen staining around adipocytes. 3C: 3 weeks post-treatment showing positive collagen staining (newly synthetized collagen) around adipocytes. 3D: Magnification of the 3 weeks post-treatment sample showing the newly synthetized collagen (arrows).

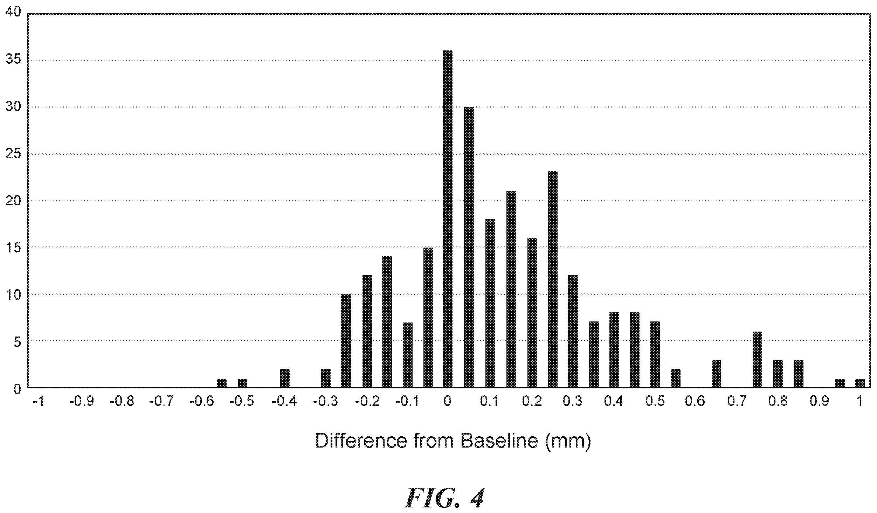

FIG. 4: Histogram of thigh skin thickness change in response to controlled epidermal cooling. Skin thickness measurements were obtained using 50 MHz ultrasound at 270 target sites across 20 human subjects by using the manufacturer's companion analysis software. Histogram shows the distribution of differences between baseline thickness and thickness 12 weeks after the final treatment across all 270 target sites.

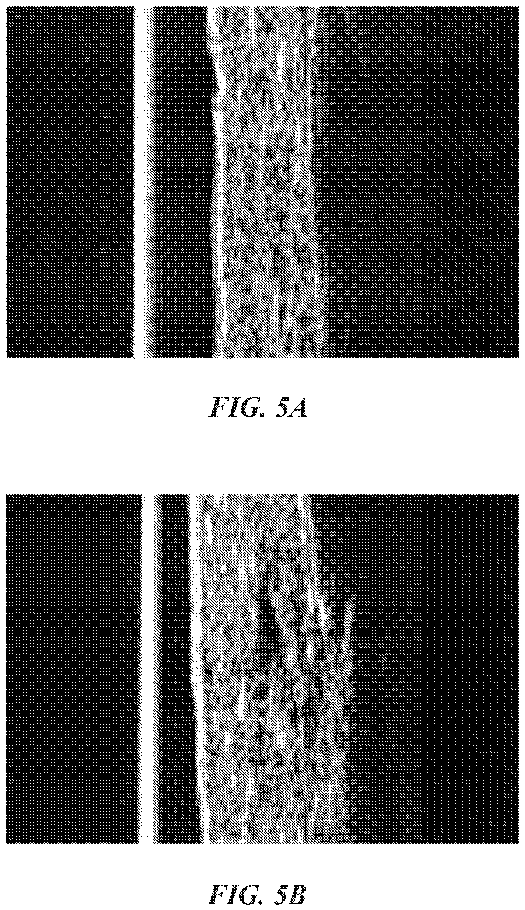

FIGS. 5A-5H: Representative images of the effect of controlled epidermal cooling on skin thickness in two subjects at two different sites. Skin is shown as a heterogeneous echogenic band at the center of the images, top bright layer is the ultrasound liner (thin hyperechoic band), and, coupling gel lies between skin and liner (markedly hypoechoic band). 5A: Subject 1, site 1, baseline (1.42 mm). 5B: Subject 1, site 1, 12 weeks after final treatment (2.05 mm). 5C: Subject 1, site 2, baseline (1.28 mm). 5D: Subject 1, site 2, 12 weeks after final treatment (1.77 mm). 5E: Subject 2, site 1, baseline (0.96 mm). 5F: Subject 2, site 1, 12 weeks after final treatment (1.19 mm). 5G: Subject 2, site 2, baseline (1.05 mm). 5H: Subject 2, site 2, 12 weeks after final treatment (1.23 mm).

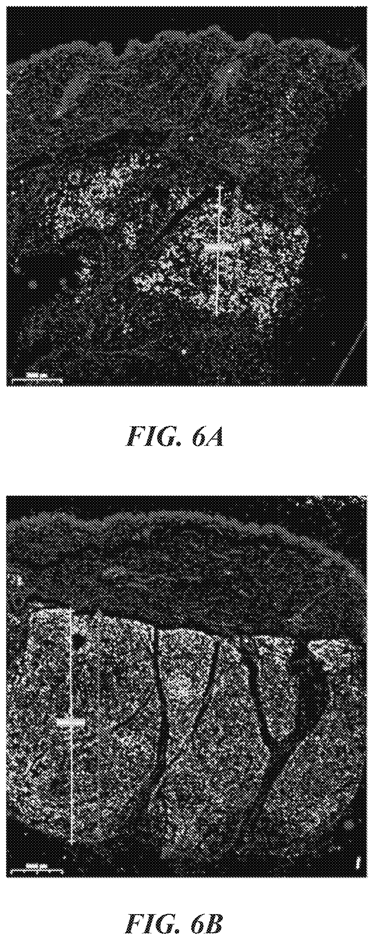

FIGS. 6A-6C: Representative tissue sections showing the effect of different treatment durations of controlled epidermal cooling in the signaling depth of TGF-β mRNA in skin and adipose tissue visualized by in situ hybridization. Fluorescence pseudocolor: TGF-β mRNA = Cy5, yellow. Nucleus = TRITC, blue. 6A: A site of a subject following treatment at -11°C for 20 minutes with signaling depth into the fat about 5 millimeters (mm) of TGF-β mRNA. 6B: A site of a subject following treatment at -11°C for 35 minutes with signaling depth into the fat about 9 millimeters (mm) of TGF-β mRNA. 6C: A site of a subject following treatment at -11°C for 60 minutes with signaling depth into the fat about 14.5 millimeters (mm) of TGF-β mRNA.

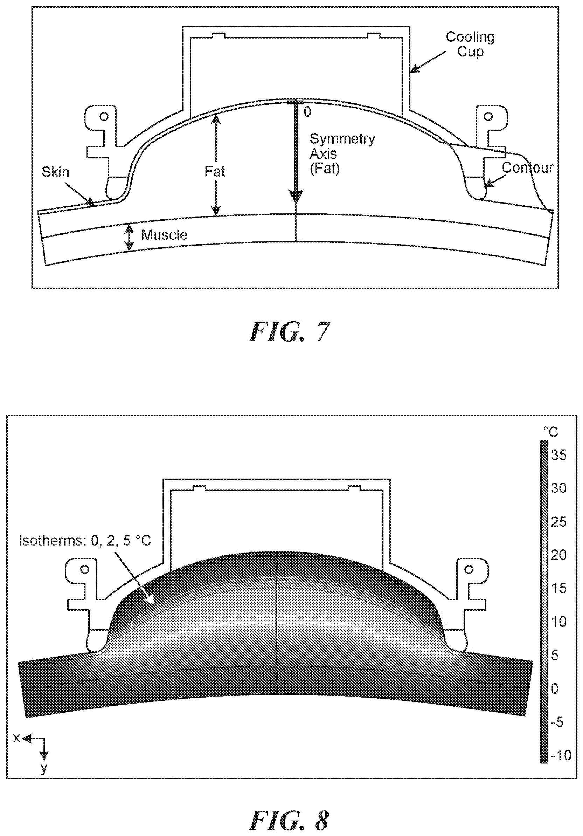

FIG. 7: Cross-sectional illustration of a computational bioheat transfer model for controlled cooling on a target treatment region showing the cooling cup, skin, adipose and muscle tissue layers.

FIG. 8: Cross-sectional view of the temperature distribution within tissue for a controlled cooling treatment at -11°C for 35 minutes (bioheat transfer model depicted in FIG.7). Colorbar indicates the temperature range in degrees Celsius. Isotherms for 0, 2, and 5 degrees Celsius are included for reference of the cooled tissue extent. The transient bioheat transfer three-dimensional model was solved using commercially available finite element analysis software (COMSOL Multiphysics v 5.0, COMSOL Inc., Burlington, MA).

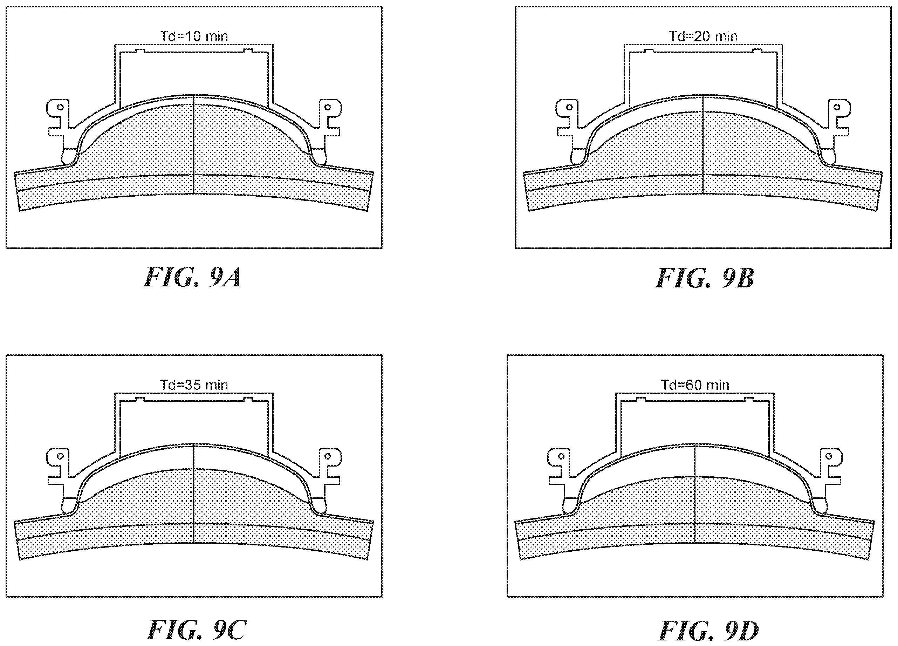

FIGS. 9A-9D: Cross-sectional view of a two-color (yellow-blue) map within tissue for a controlled cooling treatment at -11°C. Colormap is divided at a threshold temperature (Ts) of 5°C such as yellow color represents tissue at a temperature, T≤ 5°C, and dark-blue represents tissue with T >5°C. Simulations for different treatments durations (Td) are presented in 9A: Td of 10 minutes. 9B: Td of 20 minutes. 9C: Td of 35 minutes. 9D: Td of 60 minutes.

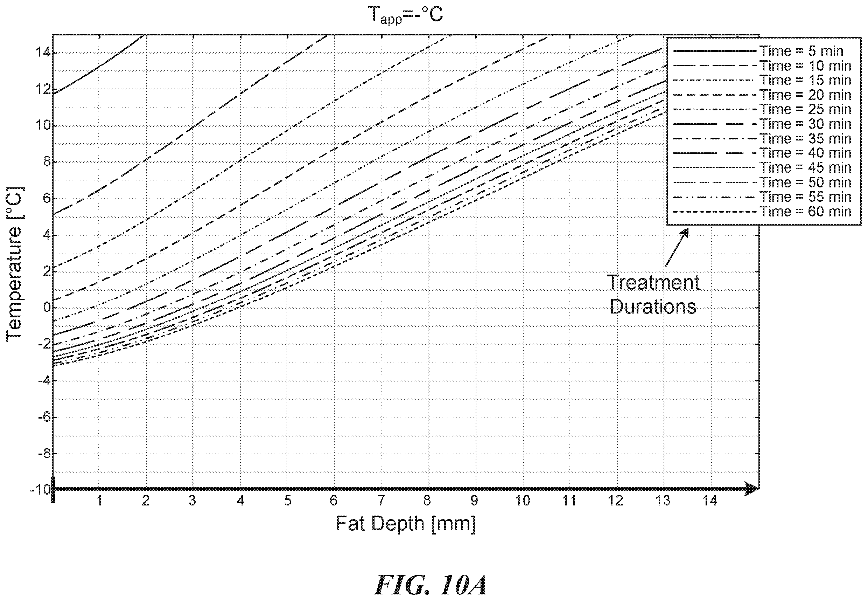

FIGS. 10A-10C: Temperature profiles along symmetry axis of the model within fat (See, Fig.7) for different treatment durations (Td) and different applied controlled cooling temperatures (Tapp). 10A: Tapp of -5°C. 10B: Tapp of -11°C. 10C: Tapp of - 15°C.

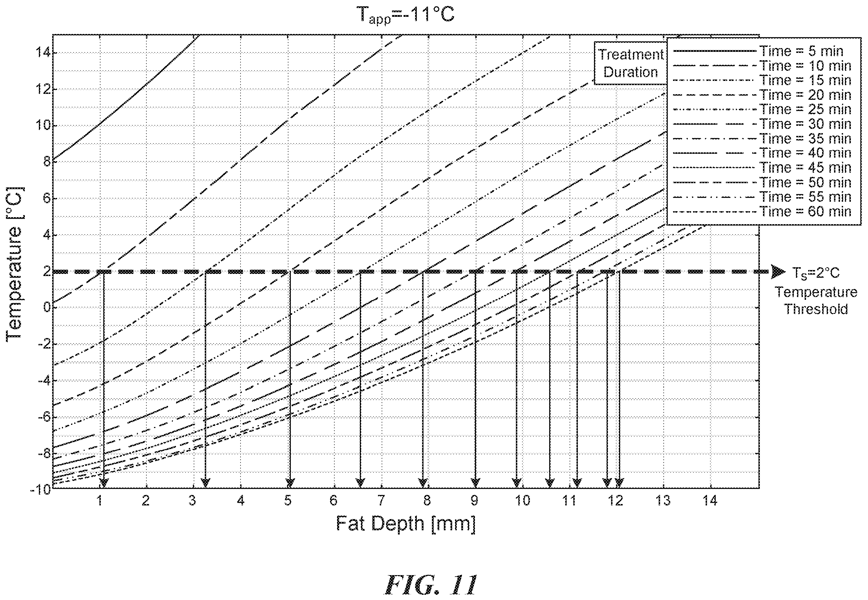

FIG. 11: Temperature profile along symmetry axis within fat for different treatment durations and a controlled cooling temperature, Tapp of -11°C. Curves were analyzed at a temperature threshold (Ts) of 2°C (dashed line). Signaling depth was assessed for different time durations (Td), arrows.

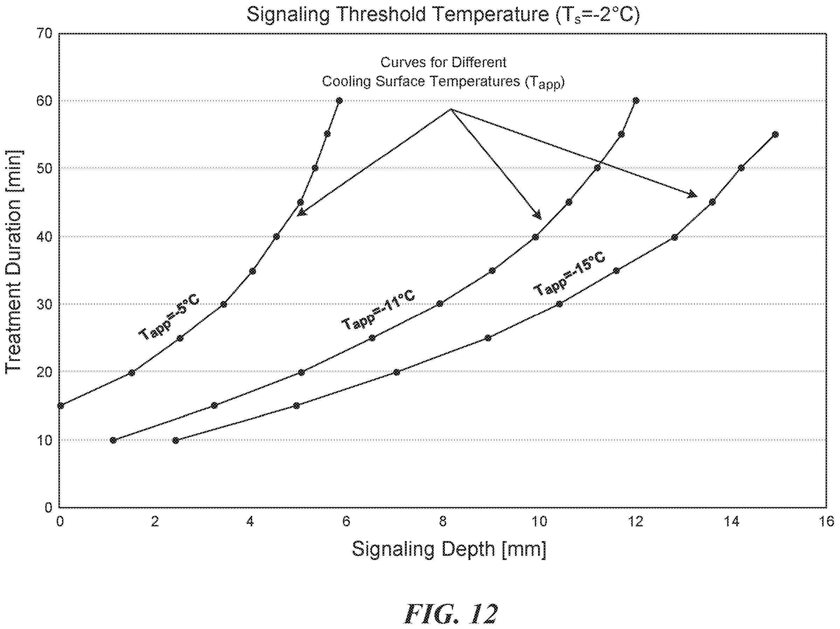

FIG. 12: Signaling depth curves at a threshold temperature of Ts of 2°C for different controlled cooling temperatures: Tapp of -5°C, -11°C (exemplary calculation shown in FIG.11), and -15°C.

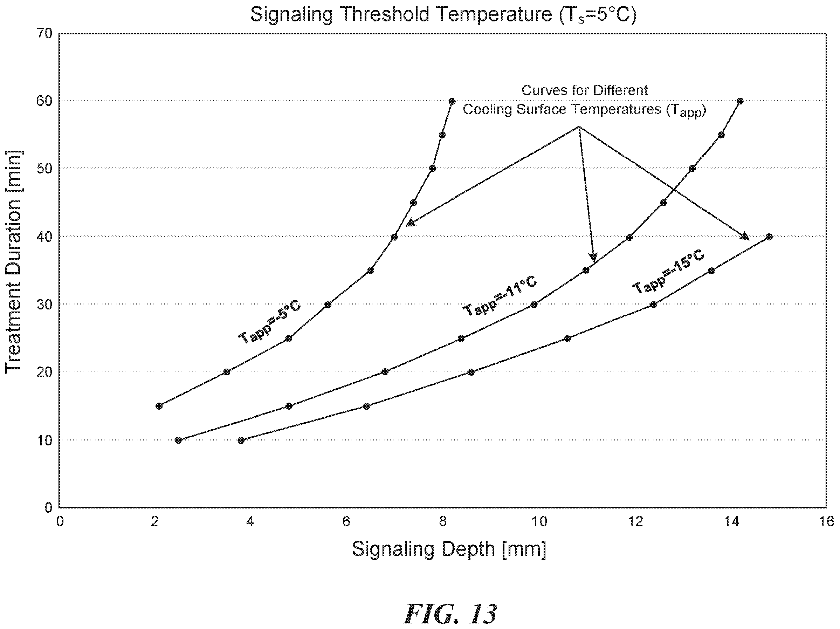

FIG. 13: Signaling depth curves at a threshold temperature of Ts of 5°C for different controlled cooling temperatures: Tapp of -5°C, -11°C, and -15°C).

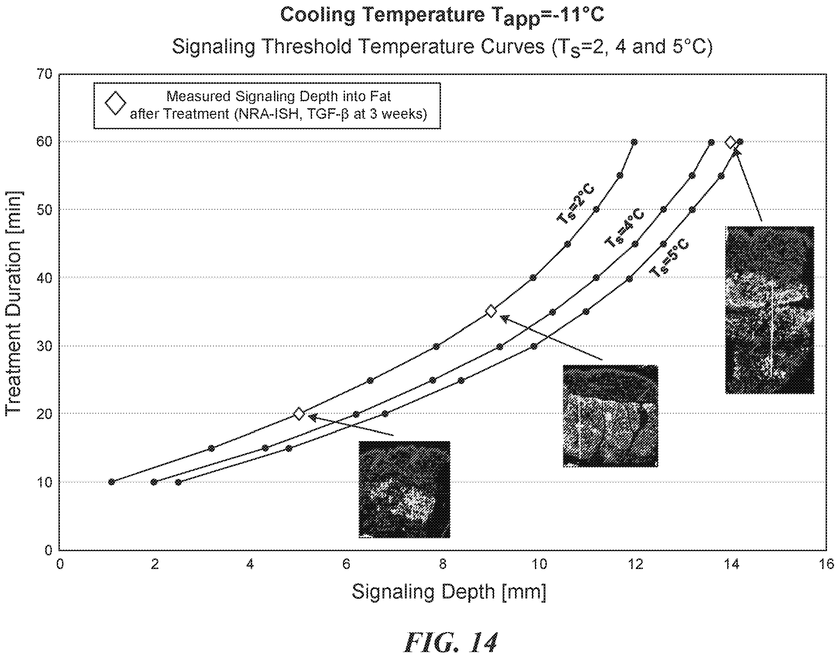

FIG. 14: Comparison of observed signaling depth (as measured in tissue sections from in vivo tests, see FIG. 6A - 6C) and theoretical signaling depth curves (from bioheat transfer model) at a Tapp of -11°C and Ts of 2°C, 4°C, and 5°C.

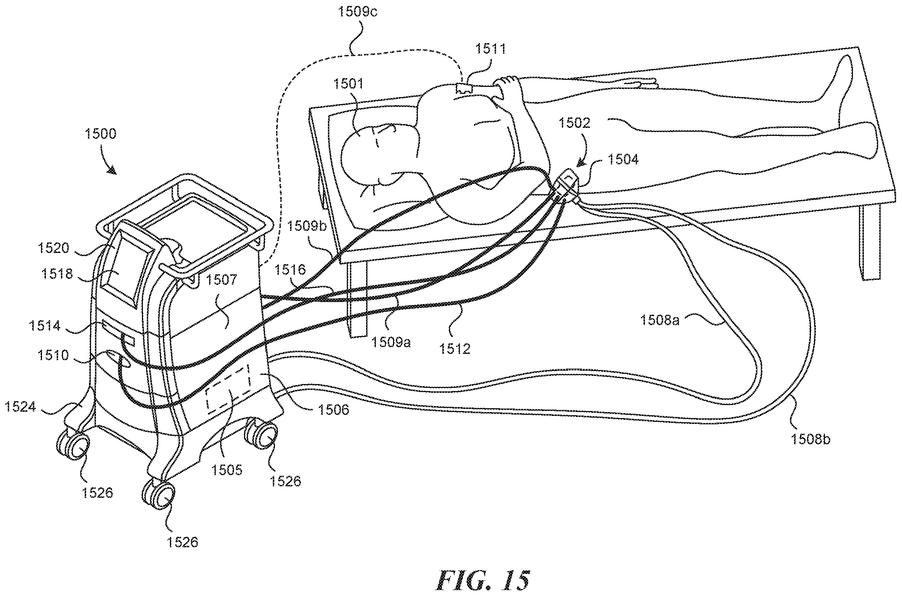

FIG. 15 is a partially schematic, isometric view of a treatment system for non-invasively removing heat from subcutaneous lipid-rich target areas of a subject in accordance with an embodiment of the technology.

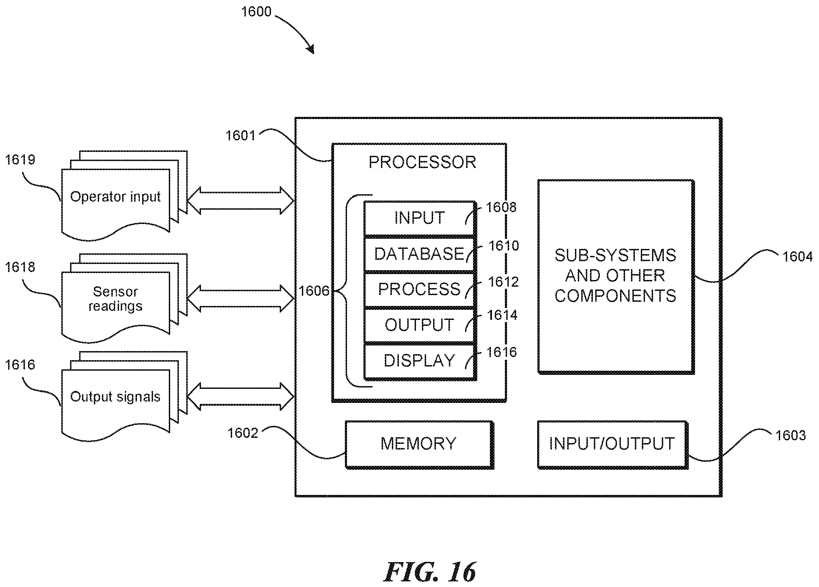

FIG. 16 is a schematic block diagram illustrating computing system software modules and subcomponents of a computing device suitable to be used in the system of FIG. 15 in accordance with an embodiment of the technology.

### DETAILED DESCRIPTION

The following description of the invention is merely intended to illustrate various embodiments of the invention. As such, the specific modifications discussed herein are not to be construed as limitations on the scope of the invention. It will be apparent to one skilled in the art that various equivalents, changes, and modifications may be made without departing from the scope of the invention, and it is understood that such equivalent embodiments are to be included herein.

Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, stages, or characteristics may be combined in any suitable manner in one or more examples of the technology. The headings provided herein are for convenience only and are not intended to limit or interpret the scope or meaning of the technology.

The methods, systems, and devices provided herein are based on the unexpected finding that epidermal cooling performed at a temperature and for a time that is insufficient to cause significant damage to underlying subcutaneous lipid-rich cells activates one or more adipocyte signaling pathways in epidermal, dermal and subcutaneous fat sufficient to cause various beneficial effects in the adjacent dermal and epidermal layers, including improvements in skin appearance that might otherwise occur when epidermal cooling is performed at a temperature and for a time sufficient to cause significant damage (e.g., damage in excess of 20%) to the underlying subcutaneous lipid-rich cells. In some embodiments, the beneficial effects on the dermal and epidermal layers (e.g., skin) occur in response to activation of the one or more adipocyte signaling pathways which result in changes to the subject's subcutaneous layer. For example, increased production of collagen in the subject's subcutaneous layer, dermal and/or epidermal layer can result in improved skin appearance.

These unexpected findings are illustrated by the experimental examples set forth below, which show that administration of controlled epidermal cooling performed at a temperature and for a time to produce a significant increase in TGF-β mRNA expression in both dermal and subcutaneous fat, with the effect on subcutaneous fat being most pronounced. As used herein, the terms "controlled cooling" or "controlled epidermal cooling" may be used interchangeably and refer to cooling of a subject's epidermis that is performed at a temperature and for a time that is insufficient to cause significant damage to underlying subcutaneous lipid-rich cells. Controlled cooling performed also produced a significant increase in collagen COL1A1 mRNA expression in fat, with a concomitant increase in collagen synthesis near treated fat tissue, such as in the subcutaneous layer and dermal and/or epidermal layers. Increased collagen production is associated with a host of beneficial aesthetic effects, including, for example, tighter, smoother skin with fewer visible lines and wrinkles or less pronounced lines and wrinkles. Based on these results, the effects of controlled epidermal cooling on skin thickness was evaluated in human subjects. Subjects exhibited a significant increase in thigh skin thickness 12 weeks following their last treatment.

The terms "controlled sub-cryolipolytic cooling" and "sub-cryolipolysis" as used herein refer to controlled cooling of the epidermis, and any concomitant cooling of the adjacent dermal and subcutaneous layers that lie beneath the epidermis being cooled, that does not result in significant damage to or destruction of subcutaneous fat cells. In other words, it does not result in damaging 20% or more of the subcutaneous fat cells.

Although certain beneficial skin effects have been observed previously in conjunction with cryolipolytic fat removal procedures, it had been assumed that these benefits were the result of significant damage to and/or destruction of subcutaneous lipid-rich cells throughout the subcutaneous layer. The results disclosed herein provide the first indication that beneficial skin effects may also be obtained using sub-cryolipolytic cooling protocols that do not damage, or that minimally damage, lipid-rich cells in the subcutaneous layer (e.g., controlled cooling). Without intending to be bound by any particular theory, it is thought that certain signaling events which occur during cryolipolysis (e.g., cooling treatments delivered by a skin surface applicator which has a temperature of about -11°C and is applied to skin for about 35 minutes) are also induced during use of sub-cryolipolytic cooling protocols. However, unlike cryolipolysis, which causes significant damage to and/or destruction of subcutaneous lipid-rich cells throughout the subject's subcutaneous layer, sub-cryolipolytic cooling protocols do not cause the same or generally similar significant damage and/or destruction. While the upper third or about the upper third of the subject's subcutaneous layer is being treated to and/or using the same, similar, or generally similar temperatures with cryolipolysis and sub-cryolipolysis, a duration of the temperature applied to the upper third of the subject's subcutaneous layer is shorter during sub-cryolipolysis compared to cryolipolysis. It is thought that the shorter durations of temperature used during sub-cryolipolysis result in reduced damage to the subject and fewer fat cells being destroyed and/or damaged compared to cryolipolysis while maintaining the same, similar, or generally similar level, amount, type, and/or degree of signaling events in the subject following sub-cryolipolytic or cryolipolytic therapy.

Provided herein in certain embodiments are methods for altering adipocyte signaling in a subject comprising cooling the subject's skin at a target site to a degree that alters adipocyte signaling but does not produce significant damage to subcutaneous lipid-rich cells. In certain embodiments, these methods result in improvements to one or more skin characteristics. Accordingly, also provided herein are methods of improving one or more skin characteristics in a subject comprising cooling the subject's skin at a target site to a degree that alters adipocyte signaling but does not produce significant damage to subcutaneous lipid-rich cells. Examples of skin characteristics that may be improved using these methods including, but are not limited to, thickness, firmness, smoothness, tightness, dermal/epidermal hydration, and collagen content. Accordingly, provided herein in certain embodiments are methods of increasing skin and/or fibrous septae thickness, increasing collagen production, increasing collagen content, increasing skin firmness, increasing skin smoothness, increasing skin tightness, and increasing dermal and/or epidermal hydration in a subject. In certain embodiments, the methods provided herein may be used for dermal remodeling, regenerative remodeling, healing skin (e.g., wound healing), or enhancing a skin healing response.

A "target site" as used herein refers to a portion of a subject's epidermis (e.g., an outer surface of the subject's skin) that is subjected to controlled cooling. In those embodiments where controlled cooling is carried out using a treatment unit (e.g., cooling unit) placed in direct contact with a subject's skin, the target site includes at least that portion of the skin that is in direct contact with the treatment unit and the skin therebeneath.

In certain of these embodiments, application of controlled cooling to the target site may generate a "treatment site" which includes the target site and a portion of the subject's body which extends radially inward from the area of contact, for example, the portion of the subject's body which comprises at least a portion of the treatment site radially extends at least about 1 mm, at least about 2 mm, at least about 3 mm, at least about 5 mm, at least about 10 mm, at least about 15 mm, at least about 20 mm, at least about 30 mm, at least about 40 mm, or at least about 50 mm from the portion of the skin that is in direct contact with the treatment unit. In other embodiments, the treatment site can include the subject's body or at least a large portion of the subject's body. In these embodiments, controlled cooling applied to the target site can activate one or more signaling pathways in the subject that may result in one or more systemic signaling events, or generally systemic signaling events.

In some embodiments, the subject's epidermis can be controllably cooled to a target temperature within a temperature range of about -40°C to about 10°C, or to a target temperature within temperature ranges of about -25°C to about 5°C, about -20°C to about 5°C, or about -15°C to about 5°C. In certain embodiments, the subject's subcutaneous layer can be cooled to the target temperature within any of the aforementioned target temperatures about 15 mm, about 10 mm, about 9 mm, about 8 mm, about 7 mm, about 6 mm, about 5 mm, about 4 mm, about 3 mm, about 2 mm, about 1 mm, or less than about 1 mm below the subject's skin (e.g., lower surface of the subject's skin). Without intending to be bound by any particular theory, it is thought that controlled cooling (e.g., sub-cryolipolytic cooling) can be achieved at any of the aforementioned depths thereby inducing one or more signaling events in the tissue that has been sub-cryolipolytically cooled. In these embodiments, one or more signaling events are not induced in tissue further below the surface of the subject's skin than any of the aforementioned depths. In some embodiments, the subject's epidermis (e.g., epidermal layer) is cooled to at least about 5°C during sub-cryolipolysis and/or cryolipolysis.

In addition to cooling the subject's epidermis to certain temperatures, the present technology can also be used to cool the subject's subcutaneous layer (e.g., subcutaneous fat layer) about 1 mm to about 20 mm below the subject's dermal layer. In some embodiments, the subject's subcutaneous layer can be cooled to about -25°C to about 20°C, or to about -15°C to about 15°C, or to about 0°C to about 15°C about 1 mm, about 2 mm, about 3 mm, about 4 mm, about 5 mm, about 6 mm, about 7 mm, about 9 mm, about 14 mm, about 18 mm, or about 20 mm below the subject's dermal layer. Prior to application of controlled cooling, the subject's subcutaneous layer at any of the above depths can be about 35°C to about 40°C, such as about 37°C. Methods of the present disclosure can, in some embodiments, include determining a subject's baseline subcutaneous temperature to at least about 20 mm below the subject's dermal layer using known technologies useful for determining subcutaneous temperature at the aforementioned depths.

Without intending to be limiting to the types of methods or parameters of the disclosed methods, example methods for determining temperatures (e.g., dermal, epidermal, and/or subcutaneous temperatures) include indirect measurements (e.g., heat transfer equations) specific for certain tissues (e.g., skin, fat, muscle), and compositions thereof, and direct measurements. In some embodiments, direct measurements are performed using one or more systems and/or devices configured to directly measure and/or determine temperatures, such as those configured to perform electrical impedance, optical, and/or crystallization measurements. Such systems can include a detector configured to extract, *inter alia*, temperature information from the epidermis, dermis, and/or fat cells as feedback to a control unit. The detected temperature information can be analyzed by control unit based on inputted properties and/or parameters. For example, the temperature of fat cells may be determined by calculation based on the temperature of the epidermis detected by detector. Thus, the treatment system may non-invasively measure the temperature of one or more fat cells. This information may then be used by a control unit for continuous feedback control of a treatment unit, for example, by adjusting the energy/temperature of a cooling/heating element and a treatment interface, thus maintaining optimal treatment temperature of target fat cells while controlling the treatment temperature and time so as to result in the surrounding epidermis and dermis not being unduly damaged. In some embodiments, the cooling/heating element can provide adjustable temperatures in the range of about - 10°C up to 42°C. An automated temperature measurement and control sequence can be repeated to maintain such temperature ranges until a procedure is complete.

It is noted that adipose tissue reduction by cooling lipid-rich cells may be even more effective when tissue cooling is accompanied by physical manipulation (e.g., massaging) of the target tissue. In accordance with an embodiment of the present invention, a treatment unit can include a tissue massaging device, such as a vibrating device and the like. Alternatively, a piezoelectric transducer can be used within the treatment unit in order to provide mechanical oscillation or movement of the cooling/heating element. The detector can include feedback devices for detecting changes in skin viscosity to monitor the effectiveness of treatment and/or to prevent any damage to surrounding tissue. For example, a vibration detecting device can be used to detect any change in the resonant frequency of the target tissue or surrounding tissue, which can indicate a change in tissue viscosity, being mechanically moved or vibrated by a vibrating device contained in the treatment unit.

To further ensure that the epidermis and/or the dermis is not damaged by cooling treatment, an optical detector/feedback device can be used to monitor the change of optical properties of the epidermis (enhanced scattering if ice formations occur); an electrical feedback device can be used to monitor the change of electric impedance of the epidermis caused by ice formation in the epidermis; and/or an ultrasound feedback device may be used for monitoring ice formation (actually to avoid) in the skin. Any such device may include signaling control unit to stop or adjust treatment to prevent or minimize skin damage.

In accordance with an embodiment of the invention, the treatment system may include a number of configurations and instruments. Algorithms that are designed for different types of procedures, configurations and/or instruments may be included for the control unit. The treatment system may include a probe controller and a probe for minimal invasive temperature measurement of fat cells. Advantageously, the probe may be capable of measuring a more accurate temperature of fat cells, thereby improving the control of the treatment unit and the effectiveness of treatment.

Controlled cooling can occur over a period of time inversely proportional to the temperature to avoid causing damage to the treatment site. For example, in some embodiments, the treatment unit is placed on the target site and cooling is applied for a time within a time range of about 10 seconds to about 2 hours. In these embodiments, a shorter time (e.g., about 10 seconds) is used when the target temperature is, for example, about -40°C and a longer time (e.g., about 2 hours) is used when the target temperature is, for example, about 10°C. In some embodiments, the target temperature is within a temperature range of about -15°C to about 0°C and the cooling is applied for about 10 minutes to about 25 minutes. In other embodiments, the target temperature is within a temperature range of about -40°C to about 0°C and the cooling is applied for about 10 seconds to about 25 minutes. The epidermal temperature can be continuously or intermittently monitored before, during, and/or after controlled cooling treatment is applied using standard temperature measurement devices, systems, and/or methods.

"Destruction" of subcutaneous lipid-rich cells and "damage" to subcutaneous lipid-rich cells are used interchangeably herein and refer to cell killing, cell disruption, and/or cell crystallization. "Significant destruction" and "significant damage" are used interchangeably herein with regard to subcutaneous lipid-rich cells and refer to destruction of less than about 20%, less than about 19%, less than about 18%, less than about 17%, less than about 16%, less than about 15%, less than about 10%, less than about 8%, less than about 7%, less than about 5%, less than about 3%, less than about 2%, less than about 1%, less than about 0.5%, or less than about 0.1% of subcutaneous lipid-rich cells in a particular population of subcutaneous lipid-rich cells (e.g., all subcutaneous lipid-rich cells within a certain distance of a target site and/or at a specific depth below the target site and/or in a specific volume of the subcutaneous lipid-rich cells beneath the target site, such as throughout the entire volume, or throughout a specific fraction of the entire volume beneath the target site). In some embodiments, controlled cooling is insufficient to cause crystallization in subcutaneous lipid-rich cells but may still cause damage or significant damage to subcutaneous lipid-rich cells.

"Adipocyte signaling" as used herein refers to any signaling pathway that is initiated by an adipocyte, involves an adipocyte, or otherwise elicits a response from an adipocyte that the adipocyte would have not otherwise elicited or been involved in had it not been a part of the signaling pathway. In addition, adipocyte signaling also refers to a passive or active cascade of events that can remain passive or active, or some combination thereof, including intermittently passive and/or intermittently active, until homeostatic conditions return. Adipocyte signaling therefore includes one or more events where, after an adipocyte has been injured, one or more chemokines or cytokines are released which attract immune cells and/or inflammatory cells (e.g., macrophages) which can ultimately release TGF-β or other cytokines. Adipocyte signaling involves one or more molecules selected from the group consisting of cytokines, chemokines, adipokines, peptides, transcription factors (e.g., transcription factors associated with expression of or one or more signaling events involving TGF-β) nucleic acids, saccharides or other sugar or carbohydrate-based molecules, and lipids. These molecules can also include salts, bases, phosphates, esters, ethers, alkyls, or any other derivatives thereof. Cytokines and other molecules involved in adipocyte signaling are sometimes referred to herein as adipocyte signaling molecules.

"Altering" adipocyte signaling as used herein means increasing or decreasing the level of one or more adipocyte signaling molecules from a pre-treatment baseline level. The increases or decreases in adipocyte signaling molecules may be observed in the dermal layer, subcutaneous fat layer, or both layers.

In certain embodiments, an alteration in adipocyte signaling may be an increase in one or more adipocyte signaling molecules (e.g., an increase in expression (a nucleic acid encoding the molecule or the molecule itself), production, and/or secretion of one or more adipocyte signaling molecules). This increase may represent a signal being "turned on," i.e., activation of a previously inactive or nearly inactive adipocyte signal, or it may simply represent a signal being upregulated versus pre-treatment levels.

In certain embodiments, an alteration in adipocyte signaling may be a decrease in one or more adipocyte signaling molecules (e.g., a decrease in expression (a nucleic acid encoding the molecule or the molecule itself), production, and/or secretion of one or more adipocyte signaling molecules). This decrease may represent a signal being "turned off" entirely (i.e., deactivation of a previously active adipocyte signal), or it may simply represent a signal being downregulated versus pre-treatment levels.

In certain embodiments, an alteration in adipocyte signaling may be an increase in one or more adipocyte signaling molecules and a simultaneous decrease in one or more different adipocyte signaling molecules.

In certain embodiments of the methods disclosed herein, one or more of the adipocyte signaling molecules being increased or decreased by a direct and/or indirect response to cooling are cytokines, adipokines, and chemokines. For example, in certain embodiments, the methods provided herein may cause an increase in expression of tumor growth factor beta ("TGF-β"), tumor necrosis factor alpha ("TNF-α"), interleukin 1 beta ("IL-1β"), interleukin 6 ("IL-6"), and monocyte chemoattractant protein 1 ("MCP-1"), leptin, adiponectin, resistin, acylation-stimulating protein, alpha 1 acid glycoprotein, pentraxin-3, IL-1 receptor antagonist, macrophage migration inhibitor factor, and serum amyloid A3 ("SAA3"). In certain embodiments, the increase or decrease in cytokine levels occurs in the subject's dermal layer, subcutaneous fat layer, or both layers. In some embodiments, one or more of the adipocyte signaling molecules is expressed, released, induced, silenced, degraded, or otherwise modified in response to one or more extrinsic processes. When hypoxic, the adipocyte can increase expression of and/or release one or more cytokines. For example, an extrinsic process includes an adipocyte in hypoxic conditions caused to or otherwise affected by a change in the subject's oxygen and/or nutrient supply, such as that provided by the subject's blood microcirculation, or due to prolonged blood vasoconstriction. Accordingly, provided herein in certain embodiments are methods of increasing cytokine (e.g., TGF-β) levels in a subject, including increasing TGF-β levels in the subject's dermal layer, subcutaneous fat layer, or both, by cooling the subject's skin at a target site to a degree sufficient to increase TGF-β levels but insufficient to produce significant destruction of subcutaneous lipid-rich cells.

In certain embodiments of the methods provided herein, one or more of the adipocyte signaling molecules being increased or decreased by a direct and/or indirect response to cooling are extracellular matrix components. For example, in certain embodiments, the methods provided herein may cause an increase in collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, and/or any molecules binding thereto, such as but not limited to, cell adhesion molecules (e.g., N-CAM, ICAM, VCAM), focal adhesion kinases, matrix metalloproteases, and Rho-kinases). In certain embodiments, these increases result in increased collagen production and/or content in the subject's dermal layer, subcutaneous fat layer, or both. In some embodiments, one or more alterations to one or more extracellular matrix components (e.g., ECM remodeling) are associated with the subject's fat cells. These alterations can result in the subject's skin feeling or have a perceived feeling of being more rigid, stiff, firm, or the like compared to how the subject's skin felt prior to treatment. However, these changes may not affect the skin itself directly but rather affect one or more structures directly or indirectly coupled to the subject's skin. In certain embodiments, ECM remodeling can have a threshold where the remodeling ends and results in a collagen matrix that is more robust (e.g., greater density, strength, and or length of collagen fibers) collagen matrix compared to the subject's collagen matrix prior to treatment.

Accordingly, provided herein in certain embodiments are methods of increasing collagen production and/or increasing collagen content in a subject by cooling the subject's skin at a target site to a degree sufficient to increase collagen production and/or increase collagen content but insufficient to produce significant destruction of subcutaneous lipid-rich cells. These methods may produce increased collagen production and/or collagen content in the dermal layer, the subcutaneous fat layer, or both.

In certain embodiments of the methods provided herein, adipocyte signaling is altered during the course of treatment only (i.e., signaling returns to around pre-treatment baseline levels at or around the time that cooling is discontinued). In other embodiments, adipocyte signaling remains altered for some period of time after cooling is discontinued. For example, adipocyte signaling may remain altered for 2 minutes, 15 minutes, 30 minutes, 1 hour, 2 hours, 3 hours, 5 hours, 8 hours, 12 hours, 16 hours, 24 hours, 36 hours, 48 hours, 3 days, 5 days, 7 days, 10 days, 15 days, 30 days, 60 days, 90 days, 120 days, 150 days, or more than 150 days after cooling is discontinued.

Also provided herein in certain embodiments are devices and systems for carrying out the disclosed methods. In certain embodiments, the methods provided herein utilize a treatment unit that is applied proximal to a target site on a subject's skin. Provided herein in certain embodiments are devices and systems comprising such a treatment unit, such as those described in greater detail with respect to FIG. 19.

In certain embodiments of the methods disclosed herein, the absence of significant destruction of subcutaneous lipid-rich cells following epidermal cooling may be a result of the subcutaneous layer not being cooled to a low enough temperature for a long enough period of time to trigger significant fat cell destruction. This may be a result of using a higher temperature for epidermal cooling than would normally be used for cryolipolysis procedures, a shorter duration of cooling than would normally be used for cryolipolysis procedures, or a combination thereof.

In certain embodiments of the methods disclosed herein, the absence of significant destruction of subcutaneous lipid-rich cells following epidermal cooling is a result of the subcutaneous layer not being cooled for a sufficient period of time to trigger fat cell destruction. In these embodiments, the subcutaneous layer may be cooled to a temperature that would result in significant destruction of subcutaneous lipid-rich cells over a long enough duration, but with cooling discontinued before said significant destruction occurs.

In certain embodiments of the methods, systems, and devices provided herein, epidermal cooling is performed by applying a treatment unit proximal to the epidermis at a target site, wherein the treatment unit does not cool the underlying tissue to a depth necessary for cryolipolysis but does so to achieve controlled sub-cryolipolytic cooling.

In certain embodiments of the methods, systems, and devices provided herein, epidermal cooling performed by applying a treatment unit proximal to the epidermis at a target site, wherein the treatment unit is set at a temperature that is insufficiently low to produce significant subcutaneous lipid-rich cell destruction. In these embodiments, the temperature of the treatment unit is higher than a temperature that would be used for cryolipolytic procedures. Because of this relatively higher temperature, application of the treatment unit does not lower the temperature of the subcutaneous layer to a degree that would result in significant subcutaneous lipid-rich cell destruction.

As described in detail above, epidermal cooling (e.g., controlled cooling) is performed by applying a treatment unit proximal to the epidermis at a target site for a period of time that is insufficient to produce significant subcutaneous lipid-rich cell destruction. In these embodiments, the period of time that the treatment unit is proximal to the epidermis is shorter than a period of time that would be used for cryolipolysis. In certain embodiments, this relatively shorter exposure time means that the treatment unit does not lower the temperature of the subcutaneous layer to a degree that results in significant subcutaneous lipid-rich cell destruction. In other embodiments, this relatively shorter exposure time means that the treatment unit lowers the temperature of the subcutaneous layer to a degree that could result in significant subcutaneous lipid-rich cell destruction, but does so for a period too short to produce said destruction. In certain embodiments, the treatment unit may be applied proximal to the epidermis for a period of time that is (e.g., 1/2, 1/4, 1/8, or 1/10 the time that would be used for cryolipolysis).

As described in detail above, in certain embodiments of the methods, systems, and devices provided herein where the treatment unit is applied proximal to the epidermis for a period of time insufficient to produce significant subcutaneous lipid-rich cell destruction, the treatment unit is set at or near a temperature that may be used for cryolipolysis. In other embodiments, the treatment unit is set at a temperature higher than a temperature that may be used for cryolipolysis (i.e., cooling is performed at both a higher temperature and for a shorter time period than would be used for cryolipolysis).

In certain embodiments of the methods, systems, and devices provided herein, multiple (i.e., two or more) cooling cycles may be utilized over the course of a single treatment session, with successive cooling cycles separated by non-cooling cycles, and preferably cycles of active re-warming. For example, in certain embodiments, a treatment unit may be applied proximal to the epidermis at a target site for a first cooling cycle, removed for a first non-cooling cycle, and then re-applied for a second cooling cycle. This process may be repeated for as many cycles as necessary to achieve a desired result. Alternatively, the treatment unit can remain applied proximal to the epidermis at the target site for all the cooling and warming/re-warming cycles, with the treatment unit having a cooling/heating element that can be precisely controlled. For example, a thermoelectric cooler could be used to cool, and then to re-warm, by simply reversing a voltage across the thermoelectric cooler. In certain embodiments, the non-cooling cycles may be a predetermined time period. In other embodiments, the non-cooling cycles may be variable. For example, in certain embodiments, the non-cooling cycle may be a time period sufficient for the temperature of the subcutaneous layer, dermal layer, or epidermis to increase back to a target temperature (e.g., back to a pre-treatment baseline temperature). In certain embodiments, the non-cooling cycles may utilize passive warming (i.e., the skin is allowed to naturally warm back to a baseline or other predetermined temperature without any intervention). In other embodiments, the non-cooling cycles may utilize activate warming to bring the epidermal temperature back to a baseline or other predetermined temperature.

In certain embodiments of the methods, systems, and devices provided herein, multiple (i.e., two or more) treatment sessions may be performed. For example, treatment sessions may be repeated as necessary to achieve or maintain a desired result. In certain embodiments, treatment sessions may be repeated at predetermined intervals (e.g., about every 2 days, about every 5 days, about every week, about every month, about every 2, 3, or 4 months) for a fixed period of time. Alternatively, treatment sessions may be repeated on an as-needed basis.

In some embodiments of the methods, systems, and devices provided herein, a temperature can be ramped from a first temperature, to a second temperature, to a third temperature, to a fourth temperature, and so on, during application of the epidermal cooling treatment to the target site. The temperature can be ramped-up (e.g., the first temperature is lower than the second temperature, which is lower than the third temperature, which is lower than the fourth temperature, and so on) or the temperature can be ramped-down (e.g., the first temperature is greater than the second temperature, which is greater than the third temperature, which is greater than the fourth temperature, and so on). In these embodiments, the temperature can be ramped over a portion of the treatment duration or across the entire treatment.

In certain embodiments of the methods disclosed herein, epidermal cooling does not significantly lower the temperature of the subcutaneous fat layer beneath a target site. In other embodiments, epidermal cooling may lower the temperature of the underlying subcutaneous fat layer, but only to a certain depth as described above. For example, in certain embodiments, the epidermal cooling does not significantly decrease the temperature of subcutaneous tissue at or below about 1 mm, about 2 mm, about 3 mm, about 4 mm, about 5 mm, 6 mm, 7 mm, about 9 mm, about 14 mm, about 18 mm, or about 20 mm below the subject's dermal layer. In one embodiment, the epidermal cooling may decrease the temperature of the underlying subcutaneous fat layer at about 5 mm to about 7 mm below the skin surface from a pre-treatment baseline temperature, while the underlying subcutaneous fat layer at about 7 mm or deeper is not cooled sufficiently from baseline. In these embodiments, the degree of cooling and/or the duration of cooling of the subcutaneous fat layer below about 5 mm to about 7 mm is insufficient to produce significant destruction of subcutaneous lipid-rich cells in these deeper layers. As described above, methods of the present disclosure include cooling the surface of the target area to about -40°C to about 10 °C for about 5 minutes to about 2 hours by placing the treatment unit on the target site and applying sub-cryolipolytic cooling. In some embodiments, the target temperature is within a temperature range of about -15°C to about 0°C and the cooling is applied for about 10 minutes to about 25 minutes. In other embodiments, the target temperature is within a temperature range of about -40°C to about 0°C and the cooling is applied for about 10 seconds to about 25 minutes. In certain embodiments, a significant decrease in the temperature of subcutaneous tissue refers to a decrease of about 1°C or more from a baseline temperature. The baseline temperature may be determined for an individual subject before the application of controlled cooling. Accordingly, in certain embodiments, the methods provided herein comprise a step of determining a baseline temperature of subcutaneous tissue at one or more specified depths.

In certain embodiments, sub-cryolipolytic controlled cooling maintains the temperature of subcutaneous tissue located at least about 5 mm to about 10 mm below the skin surface at or above a predetermined minimum temperature. For example, sub-cryolipolytic controlled cooling may maintain the temperature of subcutaneous tissue located at least about 5 mm to about 10 mm below the skin surface at or above a predetermined minimum temperature of about 20°C, about 15°C, about 10°C, about 5°C, about 4°C, about 3°C, about 2°C, about 1°C, about 0°C, or about -5°C.

In certain embodiments of the methods, systems, and devices provided herein, controlled sub-cryolipolytic cooling cools the epidermal and/or dermal layer to a lesser degree than would be associated with cryolipolytic fat removal.

Controlled sub-cryolipolytic cooling is expected to reduce the risk of adverse effects associated with cryolipolytic fat removal. For example, due to the higher temperatures and/or shorter exposure times, controlled sub-cryolipolytic cooling is expected to reduce the risk of epidermal damage, including hypo- or hyper-pigmentation.

In certain embodiments, a treatment unit for use in the methods, systems, and devices provided herein is the same as or similar to a treatment unit that would be used for cryolipolytic fat removal. In these embodiments, the treatment unit is capable of cooling the subcutaneous fat layer to a degree that would result in fat removal, but it is not used in this manner. For example, the treatment unit may be set at a higher temperature (i.e., cooled to a lesser degree) than would be used for fat removal, or it may be applied for shorter time periods or for fewer cooling cycles. An advantage of such treatment units is that they can be used for either cryolipolytic fat removal or for the sub-cryolipolytic methods provided herein.

In certain embodiments, a treatment unit for use in the methods, systems, and devices provided herein is different than a treatment unit that would be used for cryolipolytic fat removal. In certain of these embodiments, the treatment unit may be incapable of cooling the subcutaneous layer to the degree required for cryolipolytic fat removal. For example, the treatment unit may be designed such that it cannot be cooled to a degree necessary to significantly cool the subcutaneous layer. Alternatively, the treatment unit may incorporate a feedback mechanism whereby its temperature is increased when a target level of dermal cooling is reached, or when the subcutaneous layer begins to exhibit cooling. An advantage of such treatment units is that they reduce the risk of inadvertent over-cooling of the subcutaneous layer, and therefore may reduce the risk of one or more adverse events associated with low temperature cooling (e.g., hyperpigmentation, hypopigmentation, unwanted blistering, unwanted scarring, permanent undesirable alterations, skin freeze, loss of sensation (e.g., permanent and/or temporary) and disfiguring scars). In certain embodiments, the treatment methods, systems, and devices disclosed herein cause edema. In other embodiments, the treatment methods, systems, and devices disclosed herein induce a therapeutic amount of edema (e.g., an amount of edema which contributes to one or more desirable and/or beneficial effects on the subject). However, in some embodiments, the treatment methods, systems, and devices disclosed herein may cause transient local redness, bruising, and/or numbness.

In other embodiments, the treatment methods, systems, and devices disclosed herein can promote wound healing as intradermal adipocytes are known to mediate fibroblast recruitment during skin wound healing (Schmidt, B.A., Horsley, V. Intradermal adipocytes mediate fibroblast recruitment during skin wound healing (2013) Development (Cambridge), 140 (7), pp. 1517-1527). Without intending to be bound by any particular theory, restoration of the extracellular matrix associated with the subject's skin is expected to induce, promote, improve, or otherwise mediate wound healing at, within, or in tissue surrounding or otherwise associated with the subject's skin.

In addition to increased collagen production, the controlled non-cryolipolytic cooling methods provided herein may increase one or more additional components of the skin extracellular matrix, including, for example, one or more of elastin fibers, glycoproteins, and protein-polysaccharides. In those embodiments wherein the methods provided herein promote elastin formation, breakdown, and de novo synthesis (remodeling) and/or restoration of native elastin, these changes may be mediated by upregulation of tropoelastin expression in or near treated fat tissue.

Without being bound by any hypothesis, the observed changes in collagen production and skin thickness following controlled sub-cryolipolytic cooling may be a result of injured or stimulated cells (e.g., preadipocytes, adipocytes, local fibroblasts, inflammatory cells, stem cells) in subcutaneous fat releasing cytokines/growth factors (e.g., TGF-β, PDGF, bFGF, IGF), which in turn stimulate neighboring connective tissue cells in the dermal fat and skin (e.g., fibroblasts, myofibroblasts) to synthesize extracellular matrix components (e.g., collagen, elastin).

For example, TGF-β is known to be a key mediator of the expression of several connective tissue genes. TGF-β signaling is induced by ligand binding to its cognate cell membrane receptors, which are serine/threonine protein kinases. The cell membrane receptors are classed as type I or II receptors (TGFβRI and TGFβRII). The type II receptors are constitutively active. Upon ligand binding, they are brought into close proximity to type I receptors to phosphorylate and activate them. In the canonical signaling, receptor activation induces the C-terminal phosphorylation of a group of transcription factors (TFs) known as SMADs. The phosphorylated SMADs then form a complex with a co-mediator SMAD, SMAD4, that is translocated to the nucleus where it binds to gene promoters. In co-operation with different TFs and co-factors, these complexes control the transcription of hundreds of genes. By this, or other similar pathways, upon tissue controlled sub-cryolipolytic cooling and release of TGF-β, nearby fibroblasts and/or other reparative cells can proliferate and synthetize extracellular matrix components. Evidence of the regulation of collagen and elastin synthesis by skin cells in the presence of TGF-β has been studied widely [1-10].

One of ordinary skill in the art will recognize that the various embodiments described herein can be combined. For example, steps from the various methods of treatment disclosed herein may be combined in order to achieve a satisfactory or improved level of treatment.

The term "about" as used herein means within 10% of a stated value or range of values.

Referring to FIG. 15, the illustration is a partially schematic, isometric view showing one example of the treatment system 1500 for non-invasively removing heat from subcutaneous lipid-rich target areas of the patient or subject 1501, such as an abdominal area 1502 or another suitable area. The applicator 1504 can engage the target area of the subject 1501 and a treatment unit 1506 that operate together to cool or otherwise remove heat from the subcutaneous lipid-rich cells of the subject 1501. The applicator 1504 can be part of an application system, and the applicator 1504 can have various configurations, shapes, and sizes suitable for different body parts such that heat can be removed from any cutaneous or subcutaneous lipid-rich target area of the subject 1501. For example, various types of applicators may be applied during treatment, such as a vacuum applicator, a belt applicator (either of which may be used in combination with a massage or vibrating capability), and so forth. Each applicator 1504 may be designed to treat identified portions of the patient's body, such as chin, cheeks, arms, pectoral areas, thighs, calves, buttocks, abdomen, "love handles", back, breast, and so forth. For example, the vacuum applicator may be applied at the back region, and the belt applicator can be applied around the thigh region, either with or without massage or vibration. Exemplary applicators and their configurations usable or adaptable for use with the treatment system 100 variously are described in (e.g., commonly assigned U.S. Patent No. 7,854,754 and U.S. Patent Publication Nos. 2008/0077201, 2008/0077211 and 2008/0287839, incorporated herein by reference in their entirety). In further embodiments, the system 1500 may also include a patient protection device (not shown) incorporated into or configured for use with the applicator 1504 that prevents the applicator from directly contacting a patient's skin and thereby reducing the likelihood of cross-contamination between patients, minimizing cleaning requirements for the applicator. The patient protection device may also include or incorporate various storage, computing, and communications devices, such as a radio frequency identification (RFID) component, allowing, for example, use to be monitored and/or metered. Exemplary patient protection devices are described in commonly assigned U.S. Patent Publication No. 2008/0077201 incorporated herein by reference in its entirety.

In the present example, the system 1500 can also include the treatment unit 1506 and supply and return fluid lines 1508a-b between the applicator 1504 and the treatment unit 1506. A treatment unit 1506 is a device that can increase or decrease the temperature at a connected applicator 1504 that is configured to engage the subject and/or the target region of the subject. The treatment unit 1506 can remove heat from a circulating coolant to a heat sink and provide a chilled coolant to the applicator 1504 via the fluid lines 1508a-b. Alternatively, the treatment unit 1506 can circulate warm coolant to the applicator 1504 during periods of warming. In further embodiments, the treatment unit 1506 can circulate coolant through the applicator 1504 and increase or decrease the temperature of the applicator by controlling power delivery to one or more Peltier-type thermoelectric elements incorporated within the applicator. Examples of the circulating coolant include water, glycol, synthetic heat transfer fluid, oil, a refrigerant, and/or any other suitable heat conducting fluid. The fluid lines 1508a-b can be hoses or other conduits constructed from polyethylene, polyvinyl chloride, polyurethane, and/or other materials that can accommodate the particular circulating coolant. The treatment unit 1506 can be a refrigeration unit, a cooling tower, a thermoelectric chiller, or any other device capable of removing heat from a coolant. In one embodiment, the treatment unit 1506 can include a fluid chamber 1505 configured to house and provide the coolant. Alternatively, a municipal water supply (e.g., tap water) can be used in place of or in conjunction with the treatment unit 1506. In a further embodiment, the applicator 1504 can be a fluid-cooled applicator capable of achieving a desired temperature profile such as those described in U.S. Patent Application No. 13/830,027, incorporated herein by reference in its entirety. One skilled in the art will recognize that there are a number of other cooling technologies that could be used such that the treatment unit, chiller, and/or applicator need not be limited to those described herein.

The system 1500 can optionally include an energy-generating unit 1507 for applying energy to the target region, for example, to further interrogate cooled lipid-rich cells in cutaneous or subcutaneous layers via power lines 1509a-b between the applicator 1504 and the energy-generating unit 1507. In one embodiment, the energy-generating unit 1507 can be an electroporation pulse generator, such as a high voltage or low voltage pulse generator, capable of generating and delivering a high or low voltage current, respectively, through the power lines 1509a, 1509b to one or more electrodes (e.g., cathode, anode) in the applicator 1504. In other embodiments, the energy-generating unit 1507 can include a variable powered RF generator capable of generating and delivering RF energy, such as RF pulses, through the power lines 1509a, 1509b or to other power lines (not shown). In a further embodiment, the energy-generating unit 1507 can include a microwave pulse generator, an ultrasound pulse laser generator, or high frequency ultrasound (HIFU) phased signal generator, or other energy generator suitable for applying energy, for example, to further interrogate cooled lipid-rich cells in cutaneous or subcutaneous layers. In some embodiments (e.g., RF return electrode, voltage return when using a monopolar configuration, etc.), the system 1500 can include a return electrode 1511 located separately from the applicator 1504; power line 1509c (shown in dotted line) can electrically connect the return electrode 1511, if present, and the energy-generating unit 1507. In additional embodiments, the system 1500 can include more than one energy generator unit 1507 such as any one of a combination of the energy modality generating units described herein. Systems having energy-generating units and applicators having one or more electrodes are described in commonly assigned U.S.

Patent Publication No. 2012/0022518 and U.S. Patent Application Serial No. 13/830,413.

In the illustrated example, the applicator 1504 is associated with at least one treatment unit 1506. The applicator 1504 can provide mechanical energy to create a vibratory, massage, and/or pulsatile effect. The applicator 1504 can include one or more actuators, such as motors with eccentric weight, or other vibratory motors such as hydraulic motors, electric motors, pneumatic motors, solenoids, other mechanical motors, piezoelectric shakers, and so on, to provide vibratory energy or other mechanical energy to the treatment site. Further examples include a plurality of actuators for use in connection with a single applicator 1504 in any desired combination. For example, an eccentric weight actuator can be associated with one section of an applicator 1504, while a pneumatic motor can be associated with another section of the same applicator 1504. This, for example, would give the operator of the treatment system 1500 options for differential treatment of lipid-rich cells within a single region or among multiple regions of the subject 1501. The use of one or more actuators and actuator types in various combinations and configurations with an applicator 1504 may be possible.

The applicator 1504 can include one or more heat-exchanging units. Each heat-exchanging unit can include or be associated with one or more Peltier-type thermoelectric elements, and the applicator 104 can have multiple individually controlled heat-exchanging zones (e.g., between 1 and 50, between 10 and 45, between 15 and 21, approximately 100, etc.) to create a custom spatial cooling profile and/or a time-varying cooling profile. Each custom treatment profile can include one or more segments, and each segment can include a specified duration, a target temperature, and control parameters for features such as vibration, massage, vacuum, and other treatment modes. Applicators having multiple individually controlled heat-exchanging units are described in commonly assigned U.S. Patent Publication Nos. 2008/0077211 and 2011/0238051, incorporated herein by reference in their entirety.

The system 1500 can further include a power supply 1510 and a controller 1514 operatively coupled to the applicator 1504. In one embodiment, the power supply 1510 can provide a direct current voltage to the applicator 1504 to remove heat from the subject 1501. The controller 1514 can monitor process parameters via sensors (not shown) placed proximate to the applicator 1504 via a control line 1516 to, among other things, adjust the heat removal rate and/or energy delivery rate based on the process parameters. The controller 1514 can further monitor process parameters to adjust the applicator 1504 based on treatment parameters, such as treatment parameters defined in a custom treatment profile or patient-specific treatment plan, such as those described, for example, in commonly assigned U.S. Patent No. 8,275,442, incorporated herein by reference in its entirety.

The controller 1514 can exchange data with the applicator 1504 via an electrical line 1512 or, alternatively, via a wireless or an optical communication link. Note that control line 1516 and electrical line 1512 are shown in FIG. 15 without any support structure. Alternatively, control line 1516 and electrical line 1512 (and other lines including, but not limited to, fluid lines 108a-b and power lines 1509a-b) may be bundled into or otherwise accompanied by a conduit or the like to protect such lines, enhance ergonomic comfort, minimize unwanted motion (and thus potential inefficient removal of heat from and/or delivery of energy to subject 1501), and to provide an aesthetic appearance to the system 1500. Examples of such a conduit include a flexible polymeric, fabric, or composite sheath, an adjustable arm, etc. Such a conduit (not shown) may be designed (via adjustable joints, etc.) to "set" the conduit in place for the treatment of the subject 1501.

The controller 1514 can include any processor, Programmable Logic Controller, Distributed Control System, secure processor, and the like. A secure processor can be implemented as an integrated circuit with access-controlled physical interfaces; tamper resistant containment; means of detecting and responding to physical tampering; secure storage; and shielded execution of computer-executable instructions. Some secure processors also provide cryptographic accelerator circuitry. Secure storage may also be implemented as a secure flash memory, secure serial EEPROM, secure field programmable gate array, or secure application-specific integrated circuit.

In another aspect, the controller 1514 can receive data from an input device 1518 (shown as a touch screen), transmit data to an output device 1520, and/or exchange data with a control panel (not shown). The input device 1518 can include a keyboard, a mouse, a stylus, a touch screen, a push button, a switch, a potentiometer, a scanner, an audio component such as a microphone, or any other device suitable for accepting user input. The output device 1520 can include a display or touch screen, a printer, a video monitor, a medium reader, an audio device such as a speaker, any combination thereof, and any other device or devices suitable for providing user feedback.

In the embodiment of FIG. 15, the output device 1520 is a touch screen that functions as both an input device 1518 and an output device 1520. The control panel can include visual indicator devices or controls (e.g., indicator lights, numerical displays, etc.) and/or audio indicator devices or controls. The control panel may be a component separate from the input device 1518 and/or output device 1520, may be integrated with one or more of the devices, may be partially integrated with one or more of the devices, may be in another location, and so on. In alternative examples, the control panel, input device 1518, output device 1520, or parts thereof (described herein) may be contained in, attached to, or integrated with the applicator 1504. In this example, the controller 1514, power supply 1510, control panel, treatment unit 1506, input device 1518, and output device 1520 are carried by a rack 1524 with wheels 1526 for portability. In alternative embodiments, the controller 1514 can be contained in, attached to, or integrated with the multi-modality applicator 1504 and/or the patient protection device described above. In yet other embodiments, the various components can be fixedly installed at a treatment site. Further details with respect to components and/or operation of applicators 1504, treatment units 1506, and other components may be found in commonly assigned U.S. Patent Publication No. 2008/0287839.

In operation, and upon receiving input to start a treatment protocol, the controller 1514 can cause one or more power supplies 1510, one or more treatment units 1506, and one or more applicators 1504 to cycle through each segment of a prescribed treatment plan. In so doing, power supply 1510 and treatment unit 1506 provide coolant and power to one or more functional components of the applicator 1504, such as thermoelectric coolers (e.g., TEC "zones"), to begin a cooling cycle and, for example, activate features or modes such as vibration, massage, vacuum, etc.

Using temperature sensors (not shown) proximate to the one or more applicators 1504, the patient's skin, a patient protection device, or other locations or combinations thereof, the controller 1514 can determine whether a temperature or heat flux is sufficiently close to the target temperature or heat flux. It will be appreciated that while a region of the body (e.g., adipose tissue) has been cooled or heated to the target temperature, in actuality that region of the body may be close but not equal to the target temperature, e.g., because of the body's natural heating and cooling variations. Thus, although the system may attempt to heat or cool the tissue to the target temperature or to provide a target heat flux, a sensor may measure a sufficiently close temperature or heat flux. If the target temperature has not been reached, power can be increased or decreased to change heat flux to maintain the target temperature or "set-point" selectively to affect lipid-rich subcutaneous adipose tissue.

When the prescribed segment duration expires, the controller 1514 may apply the temperature and duration indicated in the next treatment profile segment. In some embodiments, temperature can be controlled using a variable other than or in addition to power.

In some embodiments, heat flux measurements can indicate other changes or anomalies that can occur during treatment administration. For example, an increase in temperature detected by a heat flux sensor can indicate a freezing event at the skin or underlying tissue (i.e., dermal tissue). An increase in temperature as detected by the heat flux sensors can also indicate movement associated with the applicator, causing the applicator to contact a warmer area of the skin, for example. Methods and systems for collection of feedback data and monitoring of temperature measurements are described in commonly assigned U.S. Patent No. 8,285,390.

The applicators 1504 may also include additional sensors to detect process treatment feedback. Additional sensors may be included for measuring tissue impedance, treatment application force, tissue contact with the applicator and energy interaction with the skin of the subject 1501 among other process parameters.

In one embodiment, feedback data associated that heat removal from lipid-rich cells in the cutaneous or subcutaneous layer can be collected in real-time. Real-time collection and processing of such feedback data can be used in concert with treatment administration to ensure that the process parameters used to alter or reduce subcutaneous adipose tissue are administered correctly and efficaciously.

Examples of the system 1500 may provide the applicator 1504, which damages, injures, disrupts, or otherwise reduces lipid-rich cells generally without collateral damage to non-lipid-rich cells in the treatment region. In general, it is believed that lipid-rich cells selectively can be affected (e.g., damaged, injured, or disrupted) by exposing such cells to low temperatures that do not so affect non-lipid-rich cells. Moreover, as discussed above, a cryoprotectant can be administered topically to the skin of the subject 1501 at the treatment site and/or used with the applicator 1504 to, among other advantages, assist in preventing freezing of the non-lipid-rich tissue (e.g., in the dermal and epidermal skin layers) during treatment to selectively interrogate lipid-rich cells in the treatment region so as to beneficially and cosmetically alter subcutaneous adipose tissue, treat sweat glands, and/or reduce sebum secretion. As a result, lipid-rich cells, such as subcutaneous adipose tissue and glandular epithelial cells, can be damaged while other non-lipid-rich cells (e.g., dermal and epidermal skin cells) in the same region are generally not damaged, even though the non-lipid-rich cells at the surface may be subject to even lower temperatures. In some embodiments, the mechanical energy provided by the applicator 104 may further enhance the effect on lipid-rich cells by mechanically disrupting the affected lipid-rich cells. In one mode of operation, the applicator 1504 may be configured to be a handheld device such as the device disclosed in commonly assigned U.S. Patent No. 7,854,754, incorporated herein by reference in its entirety.

Applying the applicator 1504 with pressure or with a vacuum type force to the subject's skin or pressing against the skin can be advantageous to achieve efficient treatment. In general, the subject 1501 has an internal body temperature of about 37°C, and the blood circulation is one mechanism for maintaining a constant body temperature. As a result, blood flow through the skin and subcutaneous layer of the region to be treated can be viewed as a heat source that counteracts the cooling of the subdermal fat. As such, cooling the tissue of interest requires not only removing the heat from such tissue but also that of the blood circulating through this tissue. Thus, temporarily reducing or eliminating blood flow through the treatment region, by means such as, e.g., applying the applicator with pressure, can improve the efficiency of tissue cooling and avoid excessive heat loss through the dermis and epidermis. Additionally, a vacuum can pull skin away from the body which can assist in cooling targeted underlying tissue.

The system 1500 (FIG. 15) can be used to perform several pre-treatment and treatment methods. Although specific examples of methods are described herein, one skilled in the art is capable of identifying other methods that the system could perform. Moreover, the methods described herein can be altered in various ways. As examples, the order of illustrated logic may be rearranged, sub-stages may be performed in parallel, illustrated logic may be omitted, other logic may be included, etc.

FIG. 16 is a schematic block diagram illustrating subcomponents of a computing device 1600 in accordance with an embodiment of the disclosure. The computing device 1600 can include a processor 1601, a memory 1602 (e.g., SRAM, DRAM, flash, or other memory devices), input/output devices 1603, and/or subsystems and other components 1604. The computing device 1600 can perform any of a wide variety of computing processing, storage, sensing, imaging, and/or other functions. Components of the computing device 1600 may be housed in a single unit or distributed over multiple, interconnected units (e.g., through a communications network). The components of the computing device 1600 can accordingly include local and/or remote memory storage devices and any of a wide variety of computer-readable media.

As illustrated in FIG. 16, the processor 1601 can include a plurality of functional modules 1606, such as software modules, for execution by the processor 1601. The various implementations of source code (i.e., in a conventional programming language) can be stored on a computer-readable storage medium or can be embodied on a transmission medium in a carrier wave. The modules 1606 of the processor can include an input module 808, a database module 1610, a process module 1612, an output module 1614, and, optionally, a display module 1616.

In operation, the input module 1608 accepts an operator input 1619 via the one or more input devices described above with respect to FIG. 15, and communicates the accepted information or selections to other components for further processing. The database module 1610 organizes records, including patient records, treatment data sets, treatment profiles and operating records and other operator activities, and facilitates storing and retrieving of these records to and from a data storage device (e.g., internal memory 1602, an external database, etc.). Any type of database organization can be utilized, including a flat file system, hierarchical database, relational database, distributed database, etc.

In the illustrated example, the process module 1612 can generate control variables based on sensor readings 1618 from sensors (e.g., temperature measurement components) and/or other data sources, and the output module 1614 can communicate operator input to external computing devices and control variables to the controller 1914 (FIG. 15). The display module 1616 can be configured to convert and transmit processing parameters, sensor readings 1618, output signals 1620, input data, treatment profiles and prescribed operational parameters through one or more connected display devices, such as a display screen, printer, speaker system, etc. A suitable display module 1616 may include a video driver that enables the controller 1614 to display the sensor readings 1618 or other status of treatment progression on the output device 1620 (FIG. 15).

In various embodiments, the processor 1601 can be a standard central processing unit or a secure processor. Secure processors can be special-purpose processors (e.g., reduced instruction set processor) that can withstand sophisticated attacks that attempt to extract data or programming logic. The secure processors may not have debugging pins that enable an external debugger to monitor the secure processor's execution or registers. In other embodiments, the system may employ a secure field programmable gate array, a smartcard, or other secure devices.

The memory 1602 can be standard memory, secure memory, or a combination of both memory types. By employing a secure processor and/or secure memory, the system can ensure that data and instructions are both highly secure and sensitive operations such as decryption are shielded from observation.

Suitable computing environments and other computing devices and user interfaces are described in commonly assigned U.S. Patent No. 8,275,442, entitled "TREATMENT PLANNING SYSTEMS AND METHODS FOR BODY CONTOURING APPLICATIONS," which is incorporated herein in its entirety by reference.

### EXAMPLES

### Example 1: Effect of Controlled Cryolipolytic Cooling on TGF-β Expression

A commercially available CoolAdvantage Petite^{™} treatment unit, available from Zeltiq Aesthetics, Inc., the assignee of the invention, was set to controlled cooling temperature of -11°C and was applied proximal to a target site on human subject's skin for a treatment duration of 35 minutes.

The effect of epidermal cooling on TGF-β mRNA expression in skin and fat layers was evaluated by RNA *in situ* hybridization (RNA-ISH) staining of formalin-fixed paraffin-embedded (FFPE) tissue samples using the Invitrogen viewRNA^{™} ISH assay protocol. The probe was human TGF-β1 gene (Thermofisher, #VA6-17264).

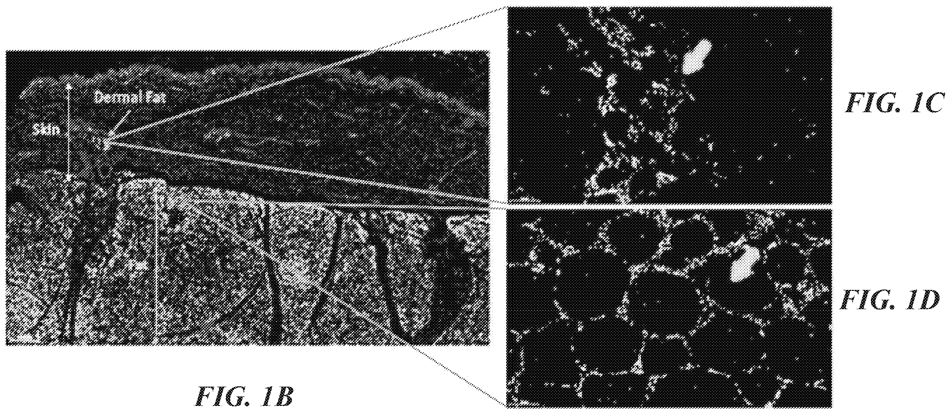

Three weeks after treatment, subjects exhibited a significant increase in TGF-β mRNA expression in fat (FIGS. 1B-1D), with little or no change in skin TGF-β mRNA levels. This increase was observed in both dermal and interfacial subcutaneous fat around adipocytes (FIG. 1C and 1D), with higher expression in the subcutaneous layer (FIG. 1B). Untreated tissue (controls) showed no expression of TGF-β mRNA in either skin or subcutaneous fat (FIG. 1A).

### Example 2: Effect of Controlled Cryolipolytic Cooling on Collagen Expression

A CoolAdvantage Petite treatment unit set to -11°C was applied proximal to a target site on human subjects' skin for 35 minutes.

The effect of epidermal cooling on collagen expression in skin and fat layers was evaluated by RNA-ISH staining of formalin-fixed paraffin-embedded (FFPE) tissue samples using the Invitrogen viewRNA^{™} ISH assay protocol. The probe was human COL1A1 (Thermofisher, #VA6-18298).

Three weeks after treatment subjects exhibited a significant increase in collagen, COL1A1 mRNA levels in subcutaneous fat tissue (compare FIGS. 2A (untreated) vs. 2B (treated)). Subcutaneous fat tissue of the untreated site showed no change or elevated signal of COL1A1 mRNA, FIG. 2A.

Upregulation of collagen mRNA is crucial for neocollagen synthesis (mRNA to protein central dogma). To ascertain whether collagen synthesis was indeed present due to mRNA upregulation following controlled sub-cryolipolytic cooling, tissue samples were stained with Masson's Trichrome (blue stain=collagen). A significant increase in fibrous collagen levels around treated adipocytes was observed following controlled sub-cryolipolytic cooling, compare FIGS. 3A (untreated) and 3B (1-week after treatment) vs. 3A (untreated) and 3C (three-weeks after treatment). A magnification of the collagen around treated adipocytes is shown in FIG. 3D. This evidence confirms the formation of neocollagen in the presence of COL1A1 and TGF-β mRNA following controlled sub-cryolipolytic cooling.

### Example 3: Effect of Controlled Sub-Cryolipolytic Cooling on Skin Thickness

A shallow surface prototype applicator (designed to conform to the thigh curvature, available from Zeltiq Aesthetics, Inc) treatment unit set to -14°C was applied proximal to a target site on 20 human subjects' skin for 20 minutes per treatment.

The effect of epidermal cooling on skin thickness was evaluated by measuring thigh skin thickness. Ultrasound were performed using a 50-MHz (DermaScan, Cortex Technology) which produces images representing the cross-section of the skin. Skin is shown as a heterogeneous echogenic band at the center of the images. Image description, layers left-to-right: Bright thin layer is the ultrasound liner (thin hyper-echoic layer), a water-based coupling transmission gel (markedly hypoechoic layer), the skin epidermis/dermis (heterogeneous echogenic band) and subcutaneous fat (markedly hypoechoic layer). Imaging was used to measure skin thickness *in-vivo* using manufacturer's analysis software. Baseline skin thickness measurements were obtained prior to treatment. Post-treatment skin thickness measurements were obtained at 12 weeks after the final treatment.

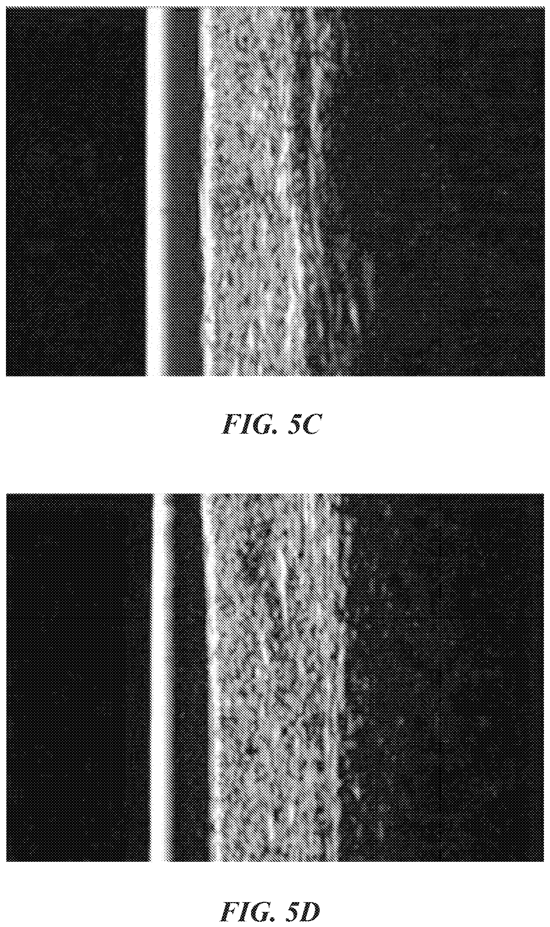

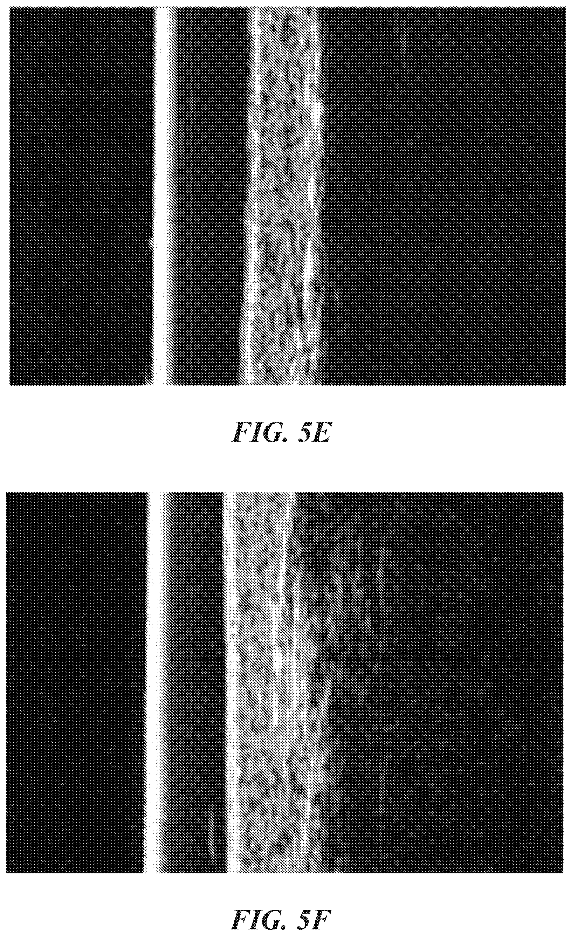

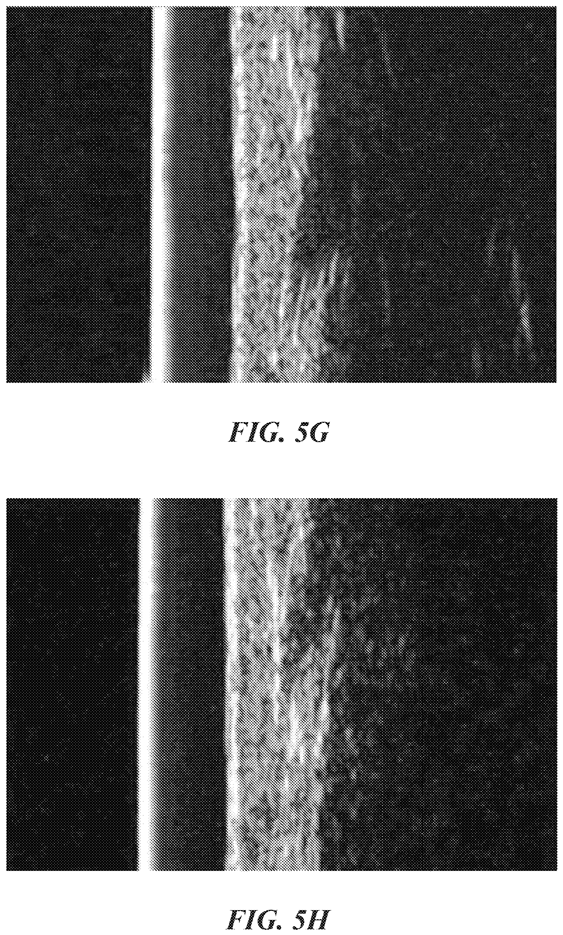

A total of 270 target sites were evaluated across the 20 subjects. Subjects exhibited a mean baseline thickness measurement of 1.45 ± 0.29 mm. At 12 weeks post-treatment, the mean thickness measurement was 1.57 ± 0.31 mm, with an overall mean increase from baseline of 0.11 ± 0.27 mm. A histogram showing the distribution of skin thickness changes across all target sites is set forth in FIG. 4. Examples of the specific changes observed in two different subjects are illustrated in FIG. 5. Subject 1 exhibited a change from 1.42 mm at baseline to 2.05 mm 12 weeks post-treatment at a first site (compare FIGS. 5A vs. 5B), and a change from 1.28 mm to 1.77 mm at a second site (compare FIGS. 5C vs. 5D). Subject 2 exhibited a change from 0.96 mm at baseline to 1.19 mm 12 weeks post-treatment at a first site (compare FIGS. 5E vs. 5F), and a change from 1.05 mm to 1.23 mm at a second site (compare FIGS. 5G vs. 5H).

### Example 4: Effect of Treatment Duration of Controlled Sub-Cryolipolytic Cooling on Signaling Depth in Target Site

A CoolAdvantage Petite treatment unit set to -11°C was applied proximal to a target site on human subjects' skin for treatment durations of 20, 35 and 60 minutes.

The effect of epidermal cooling on TGF-β mRNA expression in skin and fat layers was evaluated by RNA *in situ* hybridization (RNA-ISH) staining of formalin-fixed paraffin-embedded (FFPE) tissue samples using the Invitrogen viewRNA^{™} ISH assay protocol. The probe was human TGF-β1 gene (Thermofisher, #VA6-17264).

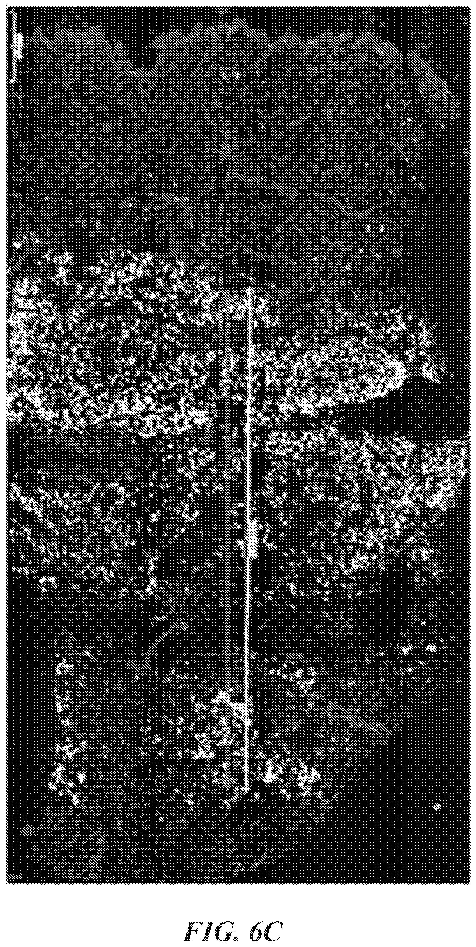

As shown in FIGS. 6A-6C, increasing the duration of treatment increases the depth of TGF-β mRNA expression into the subject's subcutaneous fat layer from about 5 mm (20-minute treatment in FIG. 6A), to about 9 mm (35-minute treatment in FIG. 6B), to about 14.5 mm (60-minute treatment in FIG. 6C).

### Example 5: Effect of Controlled Sub-Cryolipolytic Cooling on Temperature Distribution and Signaling Depth in Target Site: Theoretical and Experimental

A computational three-dimensional model of the CoolAdvantage Petite was created as shown in FIG. 7, all relevant physical boundary and initial conditions, as well as geometrical solid and tissue characteristics of the *in vivo* tests were included. Transient bioheat transfer modeling was performed using commercially available finite element analysis software (COMSOL Multiphysics v5.0 from COMSOL Inc., Burlington, MA). The bioheat transfer module was used to determine temperature distribution and depth relations as functions of cooling temperatures and treatment durations. Thermal properties and representative dimensions are shown in Table 1.

**Table 1: Summary of Tissue Thermal Properties and Thicknesses**

| ***Layer*** | ***Thickness [mm]*** | ***Density [kg*/*m^3]*** | ***Thermal conductivity [W*/*m K]*** | ***Specific Heat [J*/*kg K]*** |
|---|---|---|---|---|
| **Skin** | 2 | 1200 | 0.355 | 3350 |
| **Fat** | variable | 920 | 0.216 | 2280 |
| **Muscle** | 5 | 1270 | 0.5 | 3800 |
| **Cup (AI)** | variable | 2700 | 167 | 896 |

The data in Table 1 was adapted from Cohen, ML. Measurement of the thermal properties of human skin. A review. J. Invest. Dermatol., 69, pp. 333-338, 1977; Duck, F.A., Physical Properties of Tissues: A comprehensive Reference Book, Academic Press, 1990; and Jimenez Lozano, J.N., Vacas-Jacques, P., Anderson, R.R., Franco, W. Effect of fibrous septa in radiofrequency heating of cutaneous and subcutaneous tissues: Computational study, Lasers in Surgery and Medicine, 45 (5), pp. 326-338, 2013.

A cross-sectional view of the temperature distribution within tissue in a treatment site during application at -11°C for 35 minutes is shown in FIG. 8. Isotherms at 0, 2 and 5 °C highlight the extent of the cooled tissue at temperatures at or below those specific temperatures. For reference, we can define a threshold temperature (Ts) as the limit temperature at which signaling events are triggered (e.g. increased expression of TGF-β and/or COL1A1 mRNA). By doing so, we can map the tissue domains that enclose signaling and non-signaling tissue. Temperature thresholds may change between different cell types, molecular content, and other biological characteristics. The effect of treatment duration (Td) in the volume extent of signaling within tissue at a threshold temperature of 5°C is shown in FIGS. 9A-9D. As shown in FIGS. 9A-9D, the volume of tissue at Ts ≤ 5°C (tissue undergoing signaling) increases with the treatment duration, such as from 10 minutes (FIG. 9A), to 20 minutes (FIG. 9B), to 35 minutes (FIG. 9C), to 60 minutes (FIG. 9D) at a fixed cooling temperature (Tapp).

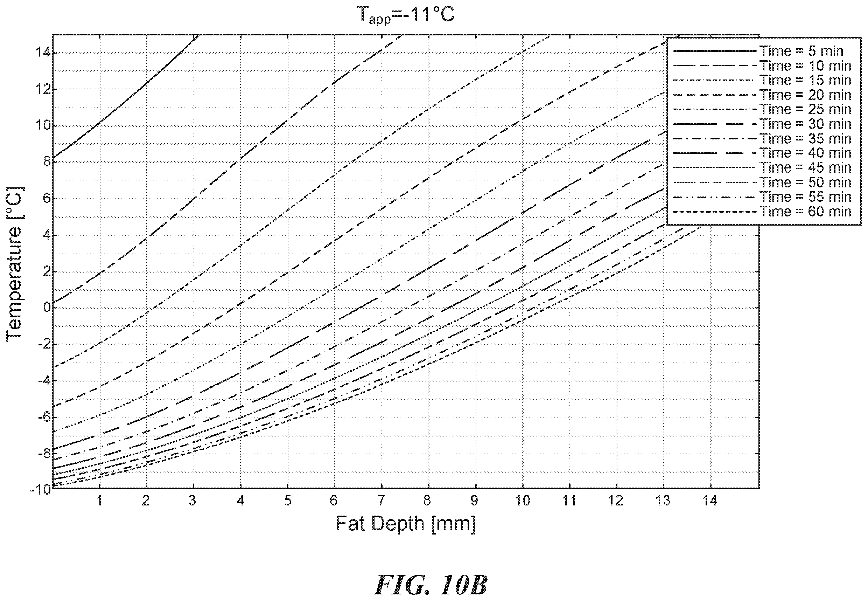

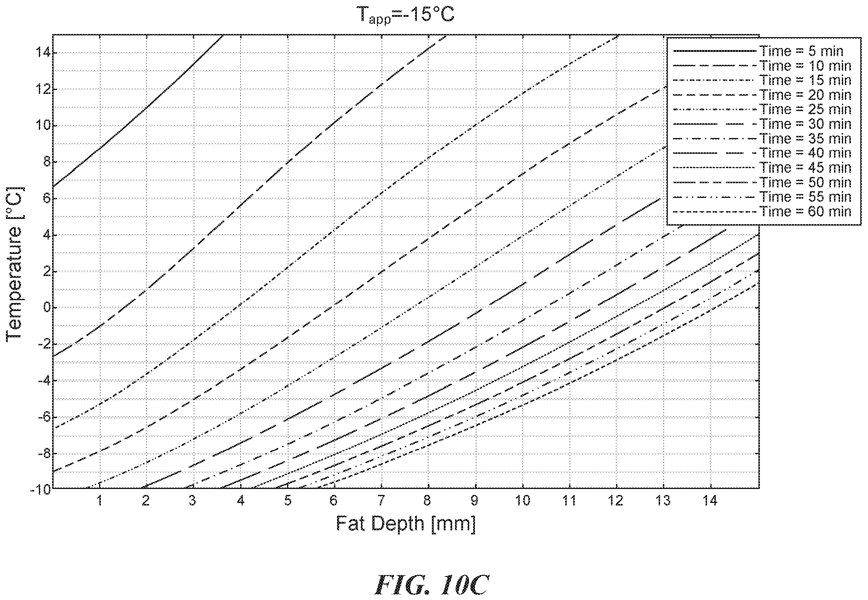

Similarly, changing Tapp can be used to vary the extent of signaling within tissue (compare FIGS. 10A-10C). These curves were calculated to quantify the maximum signaling depth into the fat (temperature profile at the symmetry line, see FIG.7) and to inspect the variation of the signaling depth for changes in Td, Tapp and Ts. Temperature profiles for varying cooling temperatures are shown in FIG. 10A (Tapp = -5 °C), FIG. 10B (Tapp = -11°C) and FIG. 10C (Tapp = -11°C) where color curves represent specific treatment durations (Td).

For fixed conditions, for example, a cooling temperature (Tapp = -11°C) and a specific threshold temperature (Ts= 2°C), the signaling depth can be evaluated for any treatment duration (Td) as shown in FIG. 11. Treatment durations of FIG.11 were set from 5 to 60 minutes and signaling fat depths were assessed (arrows) for each curve. Similar curves can be created to inspect the effect of Tapp in signaling depth for specific treatment durations (Td) at specific threshold values Ts=2°C (FIG.12) and Ts=5°C (FIG.13)

Comparison of signaling depth between theoretical values and *in vivo* tests are shown in FIG.14 for a fixed cooling temperature (CoolAdvantage Petite, Tapp = -11 °C) and different treatment durations. Curves for threshold temperatures of 2, 4 and 5°C were compared with the *in vivo* tests results presented in FIG. 6A-6C and showed a close correlation with increased expression of TGF-β1 mRNA. These outcomes represent supporting evidence that the signaling response can be controlled with the specific sub-cryolipolytic cooling conditions and methods presented herein.

As explained above, sub-cryolipolytic cooling can induce one or more signaling events in a subject; however, the parameters associated with sub-cryolipolytic cooling treatment protocols do not cause significant damage to the subject's subcutaneous layer (e.g., a volume of the subject's fat is not aesthetically decreased by at least about 20% as is observed with cryolipolytic cooling). While one or more signaling events are induced at about 2°C, about 3°C, or about 5°C, and aesthetic reduction in the subject's subcutaneous fat occurs at about 2°C, about 5°C, and about 10°C; to achieve the aesthetic fat reduction, the subject's subcutaneous fat layer is treated (e.g., cooled to either 2°C, 5°C, or 10°C) to at least about 10 mm below the surface of the subject's skin. In contrast, sub-cryolipolytic cooling is achieved by using temperatures and/or treatment times that do not significantly damage the fat in the subcutaneous layer more than about 7, 8,or 9 mm below the upper skin surface (e.g., deep subcutaneous layer fat is minimally affected).

Durations of treatment, applicator temperature, signaling threshold temperature, and thickness of the treatment area can be selected using the graphs, charts, and other illustrations as represented in FIGS. 10-14 to achieve sub-cryolipolytic cooling (e.g., induce one or more signaling events without significant destruction of subcutaneous fat). For example, if the applicator temperature is about -11°C and the signaling temperature is about 2°C, the duration of treatment needed to achieve this signaling temperature at a desired depth into the subject's sub-cutaneous layer between 1 mm and 12 mm can be determined from FIG. 11. As another example, using FIGS. 14-18 and the principles therein, one can select an applicator temperature of about -5°C to about -15°C to achieve a signaling temperature of about 2, 4, or 5°C. Also, a desired depth into the subject's sub-cutaneous layer a signaling temperature is achieved and a duration of treatment of treatment necessary to achieve this signaling depth can be determined.

In addition, a thickness of the subject's subcutaneous layer (e.g., fat layer) to be damaged can be related to a thickness of the subject's skin layer (e.g., subject's having thicker skin layers may need to have a thicker layer of fat be damaged to result in adequate signaling so that the skin can be adequately affected). As such, the parameters selected for sub-cryolipolytic cooling could also consider the subject's skin layer thickness, such that signaling depths in the subject's subcutaneous layer can be chosen to be a factor of about 0.5 times, about 1 time, about 2 times, or about 3 times thicker than the subject's skin layer. The factor can depend on a duration of time that the one or more signaling events occur in the subject's sub-cutaneous layer. For example, an applicator temperature of about -25°C can cool deeper into the subject's subcutaneous layer more rapidly than an applicator temperature of about -15°C, however, the duration of treatment could be longer at about -25°C compared to about -15°C if the one or more desired signaling events are not sufficiently otherwise induced.

### ADDITIONAL EMBODIMENTS

Various embodiments of the technology are described above. It will be appreciated that details set forth above are provided to describe the embodiments in a manner sufficient to enable a person skilled in the relevant art to make and use the disclosed embodiments. Several of the details and advantages, however, may not be necessary to practice some embodiments. Additionally, some well-known structures or functions may not be shown or described in detail, so as to avoid unnecessarily obscuring the relevant description of the various embodiments. Although some embodiments may be within the scope of the technology, they may not be described in detail with respect to the Figures. Furthermore, features, structures, or characteristics of various embodiments may be combined in any suitable manner. Moreover, one skilled in the art will recognize that there are a number of other technologies that could be used to perform functions similar to those described above. While processes or blocks are presented in a given order, alternative embodiments may perform routines having stages, or employ systems having blocks, in a different order, and some processes or blocks may be deleted, moved, added, subdivided, combined, and/or modified. Each of these processes or blocks may be implemented in a variety of different ways. Also, while processes or blocks are at times shown as being performed in series, these processes or blocks may instead be performed in parallel, or may be performed at different times. The headings provided herein are for convenience only and do not interpret the scope or meaning of the described technology.

The terminology used in the description is intended to be interpreted in its broadest reasonable manner, even though it is being used in conjunction with a detailed description of identified embodiments.

Unless the context clearly requires otherwise, throughout the description, the words "comprise," "comprising," and the like are to be construed in an inclusive sense as opposed to an exclusive or exhaustive sense; that is to say, in a sense of "including, but not limited to." Words using the singular or plural number also include the plural or singular number, respectively. Use of the word "or" in reference to a list of two or more items covers all of the following interpretations of the word: any of the items in the list, all of the items in the list, and any combination of the items in the list. Furthermore, the phrase "at least one of A, B, and C, etc." is intended in the sense that one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense that one having skill in the art would understand the convention (e.g., "a system having at least one of A, B, or C" would include but not be limited to systems that have A alone, B alone, C alone, A and B together, A and C together, B and C together, and/or A, B, and C together, etc.).

Some of the functional units described herein have been labeled as modules, in order to more particularly emphasize their implementation independence. For example, modules (e.g., modules discussed in connection with FIG. 20) may be implemented in software for execution by various types of processors. An identified module of executable code may, for instance, comprise one or more physical or logical blocks of computer instructions which may, for instance, be organized as an object, procedure, or function. The identified blocks of computer instructions need not be physically located together, but may comprise disparate instructions stored in different locations which, when joined logically together, comprise the module and achieve the stated purpose for the module.

A module may also be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

A module of executable code may be a single instruction, or many instructions, and may even be distributed over several different code segments, among different programs, and across several memory devices. Similarly, operational data may be identified and illustrated herein within modules, and may be embodied in any suitable form and organized within any suitable type of data structure. The operational data may be collected as a single data set, or may be distributed over different locations including over different storage devices, and may exist, at least partially, merely as electronic signals on a system or network.

Any patents, applications and other references cited herein are incorporated herein by reference. Aspects of the described technology can be modified, if necessary, to employ the systems, functions, and concepts of the various references described above to provide yet further embodiments.

These and other changes can be made in light of the above Detailed Description. While the above description details certain embodiments and describes the best mode contemplated, no matter how detailed, various changes can be made. Implementation details may vary considerably, while still being encompassed by the technology disclosed herein. As noted above, particular terminology used when describing certain features or aspects of the technology should not be taken to imply that the terminology is being redefined herein to be restricted to any specific characteristics, features, or aspects of the technology with which that terminology is associated.

The foregoing is merely intended to illustrate various embodiments of the present invention. The specific modifications discussed above are not to be construed as limitations on the scope of the invention. It will be apparent to one skilled in the art that various equivalents, changes, and modifications may be made without departing from the scope of the invention, and it is understood that such equivalent embodiments are to be included herein. All references cited herein are incorporated by reference as if fully set forth herein.

The following numbered aspects are also preferred embodiments of the invention, which may or may not be claimed.
1. A method of improving one or more skin characteristics in a subject comprising cooling the subject's skin at a target site to a degree that alters adipocyte signaling but does not produce significant destruction of subcutaneous lipid-rich cells, wherein said alteration of adipocyte signaling produces an improvement in one or more skin characteristics.
2. The method of aspect 1, wherein less than 10% of the subcutaneous lipid-rich cells are destroyed.
3. The method of aspect 2, wherein less than 1%, 2%, 3%, 4%, 5%, or 7% of the subcutaneous lipid-rich cells are destroyed.
4. The method of aspect 1, wherein said cooling does not produce any adverse skin effects.
5. The method of aspect 4, wherein said adverse effects are selected from the group consisting of hyper-pigmentation, hypo-pigmentation, unwanted blistering, unwanted scarring, permanent undesirable alterations, and disfiguring scars.
6. The method of aspect 1, wherein said alteration in adipocyte signaling results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3.
7. The method of aspect 6, wherein said increase in expression occurs in the dermal layer, the subcutaneous layer, or both.
8. The method of aspect 1, wherein said alteration in adipocyte signaling results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases.
9. The method of aspect 8, wherein said increase in one or more extracellular matrix components occurs in the epidermal layer, dermal layer, the subcutaneous layer, or combinations thereof.
10. The method of aspect 1, wherein said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.
11. The method of aspect 1, wherein said cooling is performed by applying a treatment unit proximal to the target site.
12. The method of aspect 11, wherein the temperature of said treatment unit is about -18°C to about 0°C.
13. The method of aspect 1, wherein said cooling lowers the temperature of the epidermis at the target site to about -15°C to about 5°C.
14. The method of aspect 11, wherein said cooling is discontinued after the temperature of the epidermis at the target site has been at a temperature of about -15°C to about 5°C for about 10 minutes to about 25 minutes.
15. The method of aspect 1, wherein said cooling does not lower the temperature of the subcutaneous fat layer 7 mm below the target site below about 3°C.
16. The method of aspect 1, wherein said cooling lowers the temperature of the subcutaneous fat layer 7 mm below the target site to about 3°C to about 30°C.
17. The method of aspect 16, wherein said cooling is discontinued after the temperature of the subcutaneous fat layer 7 mm below the target site has been at a temperature of about 3°C to about 30°C for about 10 minutes to about 25 minutes.
18. The method of aspect 1, wherein said cooling is discontinued before the temperature of the subcutaneous fat layer 7 mm below the target site falls below 3°C.
19. The method of aspect 16, wherein said cooling is repeated two or more times separated by re-warming periods during a single treatment session.
20. A method of improving one or more skin characteristics in a subject comprising:
   applying a cooling element proximal to a target site on the subject's skin for a period of time sufficient to cool the epidermis at the target site to about - 15°C to about 5°C, wherein said cooling results in an alteration of one or more adipocyte signaling events; and
   removing the cooling element before the temperature of the subcutaneous fat layer about 7 mm below the target site decreases below a temperature of +3C.
21. A method of improving one or more skin characteristics in a subject comprising:
   applying a cooling element proximal to a target site on the subject's skin for a period of time sufficient to cool the epidermis at the target site to about - 15°C to about 5°C, wherein said cooling results in an alteration of one or more adipocyte signaling events; and
   removing the cooling element before the temperature of the entire subcutaneous fat layer beneath the target site is decreased to a level that produces significant destruction of subcutaneous lipid-rich cells therein.
22. The method of aspect 21 or 22 wherein the temperature of the subcutaneous fat layer about 7 mm below the target site is decreased to about 3°C to about 15°C during application of the cooling element.
23. The method of any one of aspects 20 to 22, wherein less than 10% of the subcutaneous lipid-rich cells in the entire subcutaneous fat layer are destroyed.
24. The method of any one of aspects 20 to 23, wherein less than either 1%, 2%, 3%, 4%, 5%, or 7% of the subcutaneous lipid-rich cells are destroyed.
25. A system for improving one or more skin characteristics in a subject, comprising:
   a treatment unit; and
   an applicator having a cooling unit in communication with the treatment unit, wherein the applicator is configured to cool the subject's skin at a target site to a degree that alters adipocyte signaling but does not produce significant destruction of subcutaneous lipid-rich cells.
26. The system of aspect 25, wherein a temperature of said treatment unit is about -18°C to about 0°C.
27. The system of aspect 25 or 26, wherein, when said applicator cools the subject's skin, said cooling lowers the temperature of an epidermis at the target site to about -15°C to about 5°C.
28. The system of any one of aspects 25 to 27, wherein, when said applicator cools the subject's skin, said cooling is discontinued after the temperature of the epidermis at the target site has been at a temperature of about -15°C to about 5°C for about 10 minutes to about 25 minutes.
29. The system of any one of aspects 25 to 28, wherein, when said applicator cools the subject's skin, said cooling does not lower the temperature of the subcutaneous fat layer 7 mm below the target site below about 3°C.
30. The system of any one of aspects 25 to 29, wherein, when said applicator cools the subject's skin, said cooling lowers the temperature of the subcutaneous fat layer 7 mm below the target site to about 3°C to about 30°C.
31. The system of any one of aspects 25 to 30, wherein, when said applicator cools the subject's skin, said cooling is discontinued after the temperature of the subcutaneous fat layer 7 mm below the target site has been at a temperature of about 3°C to about 30°C for about 10 minutes to about 25 minutes.
32. The system of any one of aspects 25 to 31, wherein, when said applicator cools the subject's skin, said cooling is discontinued before the temperature of the subcutaneous fat layer 7 mm below the target site falls below 3°C.
33. The system of any one of aspects 25 to 32, wherein, when said applicator cools the subject's skin, said cooling is repeated two or more times separated by re-warming periods during a single treatment session.
34. The system of any one of aspects 25 to 33, wherein, when the applicator alters adipocyte signaling, an improvement in one or more skin characteristics is produced.
35. The system of any one of aspects 25 to 34, wherein less than 10% of the subcutaneous lipid-rich cells are destroyed.
36. The system of any one of aspects 25 to 35, wherein less than 1%, 2%, 3%, 4%, 5%, or 7% of the subcutaneous lipid-rich cells are destroyed.
37. The system of any one of aspects 25 to 36, wherein, when the applicator cools the subject's skin at the target site, the cooling does not produce any adverse skin effects.
38. The system of any one of aspects 25 to 37, wherein said adverse effects are selected from the group consisting of hyper-pigmentation, hypo-pigmentation, unwanted blistering, unwanted scarring, permanent undesirable alterations, and disfiguring scars.
39. The system of any one of aspects 25 to 38, wherein, when the applicator alters adipocyte signaling, said alteration results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3.
40. The system of any one of aspects 25 to 39, wherein, when the applicator alters adipocyte signaling, said increase in expression occurs in the dermal layer, the subcutaneous layer, or both.
41. The system of any one of aspects 25 to 40, wherein, when the applicator alters adipocyte signaling, said alteration results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases.
42. The system of any one of aspects 25 to 41, wherein, when the applicator alters adipocyte signaling, said increase in one or more extracellular matrix components occurs in the epidermal layer, dermal layer, the subcutaneous layer, or combinations thereof.
43. The system of any one of aspects 25 to 42, wherein, when the applicator alters adipocyte signaling, said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.

## Claims

1. A method of improving one or more skin characteristics in a subject comprising cooling the subject's skin at a target site to a degree that alters adipocyte signaling but does not produce significant destruction of subcutaneous lipid-rich cells, wherein said alteration of adipocyte signaling produces an improvement in one or more skin characteristics.

2. The method of claim 1, wherein less than 10% of the subcutaneous lipid-rich cells are destroyed, wherein preferably less than 1%, 2%, 3%, 4%, 5%, or 7% of the subcutaneous lipid-rich cells are destroyed.

3. The method of claim 1, wherein said cooling does not produce any adverse skin effects, wherein preferably said adverse effects are selected from the group consisting of hyper-pigmentation, hypo-pigmentation, unwanted blistering, unwanted scarring, permanent undesirable alterations, and disfiguring scars.

4. The method of claim 1, wherein said alteration in adipocyte signaling results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3, wherein preferably said increase in expression occurs in the dermal layer, the subcutaneous layer, or both.

5. The method of claim 1, wherein said alteration in adipocyte signaling results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases, wherein preferably said increase in one or more extracellular matrix components occurs in the epidermal layer, dermal layer, the subcutaneous layer, or combinations thereof.

6. The method of claim 1, wherein said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.

7. The method of claim 1, wherein said cooling is performed by applying a treatment unit proximal to the target site.

8. The method of claim 7, wherein the temperature of said treatment unit is about -18°C to about 0°C.

9. The method of claim 1, wherein said cooling lowers the temperature of the epidermis at the target site to about -15°C to about 5°C.

10. The method of claim 7, wherein said cooling is discontinued after the temperature of the epidermis at the target site has been at a temperature of about -15°C to about 5°C for about 10 minutes to about 25 minutes.

11. The method of claim 1, wherein said cooling does not lower the temperature of the subcutaneous fat layer 7 mm below the target site below about 3°C.

12. The method of claim 1, wherein said cooling lowers the temperature of the subcutaneous fat layer 7 mm below the target site to about 3°C to about 30°C.

13. The method of claim 12, wherein said cooling is discontinued after the temperature of the subcutaneous fat layer 7 mm below the target site has been at a temperature of about 3°C to about 30°C for about 10 minutes to about 25 minutes.

14. The method of claim 1, wherein said cooling is discontinued before the temperature of the subcutaneous fat layer 7 mm below the target site falls below 3°C.

15. The method of claim 12, wherein said cooling is repeated two or more times separated by re-warming periods during a single treatment session.

16. A method of improving one or more skin characteristics in a subject comprising:
applying a cooling element proximal to a target site on the subject's skin for a period of time sufficient to cool the epidermis at the target site to about - 15°C to about 5°C, wherein said cooling results in an alteration of one or more adipocyte signaling events; and
removing the cooling element before the temperature of the subcutaneous fat layer about 7 mm below the target site decreases below a temperature of +3C.

17. A method of improving one or more skin characteristics in a subject comprising:
applying a cooling element proximal to a target site on the subject's skin for a period of time sufficient to cool the epidermis at the target site to about - 15°C to about 5°C, wherein said cooling results in an alteration of one or more adipocyte signaling events; and
removing the cooling element before the temperature of the entire subcutaneous fat layer beneath the target site is decreased to a level that produces significant destruction of subcutaneous lipid-rich cells therein.

18. The method of claim 16 or 17 wherein the temperature of the subcutaneous fat layer about 7 mm below the target site is decreased to about 3°C to about 15°C during application of the cooling element.

19. The method of any one of claims 16 to 18, wherein less than 10% of the subcutaneous lipid-rich cells in the entire subcutaneous fat layer are destroyed.

20. The method of any one of claims 16 to 19, wherein less than either 1%, 2%, 3%, 4%, 5%, or 7% of the subcutaneous lipid-rich cells are destroyed.

21. A system for improving one or more skin characteristics in a subject, comprising:
a treatment unit; and
an applicator having a cooling unit in communication with the treatment unit, wherein the applicator is configured to cool the subject's skin at a target site to a degree that alters adipocyte signaling but does not produce significant destruction of subcutaneous lipid-rich cells.

22. The system of claim 21, wherein a temperature of said treatment unit is about -18°C to about 0°C.

23. The system of claim 21 or 22, wherein, when said applicator cools the subject's skin, said cooling lowers the temperature of an epidermis at the target site to about -15°C to about 5°C, wherein preferably, when said applicator cools the subject's skin, said cooling is discontinued after the temperature of the epidermis at the target site has been at a temperature of about -15°C to about 5°C for about 10 minutes to about 25 minutes.

24. The system of any one of claims 21 to 23, wherein, when said applicator cools the subject's skin, said cooling does not lower the temperature of the subcutaneous fat layer 7 mm below the target site below about 3°C.

25. The system of any one of claims 21 to 24, wherein, when said applicator cools the subject's skin, said cooling lowers the temperature of the subcutaneous fat layer 7 mm below the target site to about 3°C to about 30°C, wherein preferably, when said applicator cools the subject's skin, said cooling is discontinued after the temperature of the subcutaneous fat layer 7 mm below the target site has been at a temperature of about 3°C to about 30°C for about 10 minutes to about 25 minutes.

26. The system of any one of claims 21 to 25, wherein, when said applicator cools the subject's skin, said cooling is discontinued before the temperature of the subcutaneous fat layer 7 mm below the target site falls below 3°C.

27. The system of any one of claims 21 to 26, wherein, when said applicator cools the subject's skin, said cooling is repeated two or more times separated by re-warming periods during a single treatment session.

28. The system of any one of claims 21 to 27, wherein, when the applicator alters adipocyte signaling, an improvement in one or more skin characteristics is produced.

29. The system of any one of claims 21 to 28, wherein less than 10% of the subcutaneous lipid-rich cells are destroyed, wherein preferably less than 1%, 2%, 3%, 4%, 5%, or 7% of the subcutaneous lipid-rich cells are destroyed.

30. The system of any one of claims 21 to 29, wherein, when the applicator cools the subject's skin at the target site, the cooling does not produce any adverse skin effects., wherein preferably said adverse effects are selected from the group consisting of hyper-pigmentation, hypo-pigmentation, unwanted blistering, unwanted scarring, permanent undesirable alterations, and disfiguring scars.

31. The system of any one of claims 21 to 30, wherein, when the applicator alters adipocyte signaling, said alteration results in an increase in expression of one or more cytokines selected from the group consisting of TGF-β, TNF-α, IL-1β, IL-6, MCP-1, leptin, adiponectin, resistin, acylation-stimulating protein, alpha 1 acid glycoprotein, pentraxin-3, IL-1 receptor antagonist, macrophage migration inhibitor factor, and SAA3, wherein preferably, when the applicator alters adipocyte signaling, said increase in expression occurs in the dermal layer, the subcutaneous layer, or both.

32. The system of any one of claims 21 to 31, wherein, when the applicator alters adipocyte signaling, said alteration results in an increase in one or more extracellular matrix components selected from the group consisting of collagen, elastin, proteoglycans (e.g., heparan sulfate, keratin sulfate, and chondroitin sulfate), fibrinogen, laminin, fibrin, fibronectin, hyaluronan, hyaluronic acid, versican, aggrecan, lumican, decorin, glypican, tenascins, syndecans, integrins, discoidin domain receptors, perlecan, N-CAM, ICAM, VCAM, focal adhesion kinases, matrix metalloproteases, and Rho-kinases, wherein preferably, when the applicator alters adipocyte signaling, said increase in one or more extracellular matrix components occurs in the epidermal layer, dermal layer, the subcutaneous layer, or combinations thereof.

33. The system of any one of claims 21 to 32, wherein, when the applicator alters adipocyte signaling, said one or more improved skin characteristics are selected from the group consisting of increased skin thickness, increased new collagen content, increased skin firmness, increased skin smoothness, skin tightening, increased dermal/epidermal hydration, dermal remodeling, and fibrous septae thickening.
